# EUROPEAN PATENT APPLICATION

(11) **EP 4 276 175 A1**
(43) Date of publication of application: **15.11.2023**
(21) Application number: 21909421.6
(22) Date of filing: 21.12.2021
(51) Int. Cl.: C12N 7/01, C12N 7/04, C12N 5/10, A61K 39/12, A61P 31/14, C12R 1/93

(54) **ATTENUATED VIRUS OF FLAVIVIRUS VIRUS AND USE THEREOF**

(30) Priority: 22.12.2020 CN 202011528256
(71) Applicant: Hubei Zhangjun Biotech Co.,Ltd., Wuhan, Hubei 400070 (CN)
(72) Inventor: ZHANG, Bo, Wuhan, Hubei 430071 (CN); YE, Hanqing, Wuhan, Hubei 430071 (CN); ZHANG, Yanan, Wuhan, Hubei 430071 (CN); LI, Na, Wuhan, Hubei 430071 (CN); ZHANG, Qiuyan, Wuhan, Hubei 430071 (CN); DENG, Chenglin, Wuhan, Hubei 430071 (CN); YUAN, Shaopeng, Beijing 100036 (CN); ZHAN, Shunli, Beijing 100036 (CN); GAO, Lei, Beijing 100036 (CN)
(74) Representative: Elzaburu S.L.P.
(86) International application number: PCT/CN2021/140236
(87) International publication number: WO 2022/135425

(57) **Abstract**

Provided are an attenuated virus of a flavivirus virus and the use thereof. The attenuated virus comprises a polyadenylic acid (poly(A)) sequence, wherein the polyadenylic acid (poly(A)) is used for replacing a part of the nucleotide sequence of a 3' untranslated region (3'UTR) of the flavivirus virus, so that the 3' untranslated region (3'UTR) of the attenuated virus obtained after the part of the nucleotide sequence of the flavivirus virus is replaced at least retains a 3'-end stem loop region (3'SL). The attenuated virus can be used for preparing safe and effective attenuated vaccine strains.

## Description

### TECHNICAL FIELD

The present disclosure belongs to the field of biotechnology, and in particular relates to an attenuated virus of a flavivirus virus and use thereof.

### BACKGROUND

The flaviviruses belong to a flaviviridae family and contain more than 70 viruses, most of which are spread by mosquitoes or ticks. Among these viruses, Japanese encephalitis virus (JEV), tick-borne encephalitis virus (TBEV), yellow fever virus (YFV), West Nile virus (WNV), St. Louis encephalitis virus (SLEV), Murray Valley encephalitis virus (MVEV), dengue virus (DENV) and zika virus (ZIKV) are important human pathogens causing high morbidity and mortality worldwide. Severe flavivirus infection diseases include hemorrhagic fevers (YFV and DENV), encephalitis, and neurological sequelae (JEV, TBEV, WNV, SLEV, and MVEV), which can lead to death, with mortality from JEV, YFV, TBEV, and DENV ranging from 5% to 30%.

As shown by the World Health Organization, DENV is currently epidemic in more than 100 countries, causing 20,000 deaths each year; JEV is epidemic in Southeast Asia and the Western Pacific, with 68,000 cases reported annually; WNV broke out in New York in 1999, causing 9,862 infected cases and 264 deaths (CDC statistics), and rapidly spread from North America to Central America and finally to South America, where the epidemic area is still expanding; and following a small epidemic in Yap, Micronesia and Gabon in 2007, an unexpected large-scale outbreak of ZIKV happened in Latin America in 2016, and such ZIKV infection was associated with severe neurological disorders (Guillain-Barre syndrome) and neonatal head malformations, and thus was declared a "Public Health Emergency of Global Concern". In 2016, about 4 million cases of ZIKV infections were recorded in the United States alone. Since then, ZIKV has spread rapidly to French Polynesia, South America, Africa, Asia and other regions. As a result, flavivirus infections have become one of the major public health problems facing the world.

At present, there are no effective antiviral drugs and specific treatments against flavivirus infections, and there are only three flavivirus vaccines for human prophylaxis. Therefore, the development of novel and more effective flavivirus vaccines is of great strategic significance for the prevention of flavivirus infections in China and even the world. Among many types of vaccines, live attenuated vaccines have been focused onvaccine research and development because of their strong immunogenicity, as well as the characteristics that a single immunization can induce humoral and cellular immune responses, and induce more comprehensive and durable immune protection.

A flavivirus genome is a single-stranded positive-sense RNA with a total length of about 11,000 bases, consisting of a 5' untranslated region (5'UTR), a 3' untranslated region (3'UTR), and a long open reading frame in the middle. Although the 5' end of the genome has a 7-methylguanosine cap (m⁷GpppAmp Cap) structure similar to mRNA, the 3' end of the genome does not have a polyadenosine tail poly(A) and instead contains a conserved 5'-CU_{OH}-3' sequence. The untranslated region (UTR) of the flavivirus genome contains conserved RNA sequences that form highly complex secondary and tertiary structures, which play a multifaceted role in virus replication, translation, propagation, and pathogenicity (Viruses. 2014 Jan 27; 6(2):404-27).

The 5'UTR has a length of about 100 bases and contains: i) a conserved stem loops (SL) structure, in which SL-A is a promoter element for RNA synthesis, which binds to NS5-RdRp (Genes Dev. 2006 Aug 15; 20(16): 2238-49) and initiates the synthesis of negative-stranded RNA; and ii) cyclization sequences upstream of an initiation codon AUG (5'upstream of AUG region (5'UAR), 5' cyclization sequences (5'CS) and 5' downstream of AUG region (5'DAR)), which mediate long-range RNA-RNA interactions (Virus Res. 2009 Feb; 139(2): 230-9). The 3'UTR is long, about 400-700 bases, forming a plurality of conserved RNA secondary structures, which play an important role in the replication, pathogenicity and propagation of viruses. 3'UTR may be structurally divided into i) a stem loop region (SL); ii) a dumbbell region (DB); and iii) a short hairpin 3' stem loop region (sHP-3'SL). sHP-3'SL is a most conserved RNA secondary structure in flaviviruses, and its complementary cyclization sequences 3'CS, 3'UAR and 3'DAR are complementarily paired with corresponding 5'CS, 5'UAR and 5'DAR, resulting in genomic cyclization that is a necessary cis-action for RNA replication initiation. The stem loop region (SL) and the dumbbell region (DB), as enhancers in the genome replication process, are not very conserved in flaviviruses, and their sizes and structures vary as different viruses. Although the stem loop region (SL) and the dumbbell region (DB) play unnecessary roles in genome replication, a number of studies based on the weakening or enhancement of virulence due to different degrees of deletion or point mutation in SL and DB regions have shown that the stem loop region (SL) and the dumbbell region (DB) play important roles in regulating virus virulence, host immune response, host adaptation and the like. Therefore, the stem loop region (SL) and the dumbbell region (DB) have become two important targets for designing attenuated vaccines.

Taking overall consideration of these factors, alternative attenuated vaccine candidates of flaviviruses with improved efficacy and safety profiles are required.

### SUMMARY OF THE INVENTION

According to the present disclosure, an attenuated poly(A) virus strain of a flavivirus virus is constructed by replacing all or a part of a nucleotide sequence of a 5'-end stem loop region (SL), all or a part of a nucleotide sequence of a cyclization sequence region (CS), and/or all or a part of a nucleotide sequence of a dumbbell region (DB) in a 3' untranslated region (3'UTR) with a poly(A) sequence; and the attenuated poly(A) virus strain has been well documented as an attenuated vaccine candidate strain of a wild-type virus through extensive in vivo studies. This virus provides the basis for a vaccine that is used safely. One object of the present disclosure is to provide an attenuated flavivirus virus having attenuated properties while still having good immunogenicity. The attenuated flavivirus virus comprises a polyadenylic acid (poly(A)) sequence, wherein the poly(A) sequence is used for replacing a part of nucleotide sequence of a 3' untranslated region (3'UTR) of the flavivirus virus, so that the 3' untranslated region (3'UTR) of the attenuated flavivirus virus obtained in response to the part of the nucleotide sequence of the flavivirus virus being replaced at least retains a 3'-end stem loop region (3'SL).

Another object of the present disclosure is to provide a DNA, which can be transcribed to produce an RNA genome of the attenuated flavivirus virus as described above.

Another object of the present disclosure is to provide a cell, which comprises the attenuated flavivirus virus or the DNA as described above.

Another object of the present disclosure is to a vaccine, which comprises the attenuated flavivirus virus, the DNA and/or the cell as described above.

Another object of the present disclosure is to provide a pharmaceutical composition, which comprises the attenuated flavivirus virus, the DNA, the cell and/or the vaccine as described above, and a pharmaceutically acceptable vector.

Another object of the present disclosure is to provide a method for preparing the attenuated flavivirus virus as described above, the method comprising substituting all or a part of nucleotide sequence of a 3' untranslated region (3'UTR) of a wild-type virus with a polyadenylic acid (poly(A)) sequence.

Another object of the present disclosure is to provide use of the attenuated flavivirus virus, the DNA and/or the cell as described above in the preparation of an attenuated flavivirus virus vaccine.

Another object of the present disclosure is to provide use of the attenuated flavivirus virus, the DNA, the cell, the vaccine and/or the pharmaceutical composition as described above in the preparation of a medicament for treating or preventing diseases caused by a flavivirus virus.

Another object of the present disclosure is to provide a method for stimulating an immune response to a flavivirus virus, the method comprising immunological administration of the attenuated flavivirus virus, the DNA, the cell, the vaccine and/or the pharmaceutical composition.

### BRIEF DESCRIPTION OF THE DRAWINGS

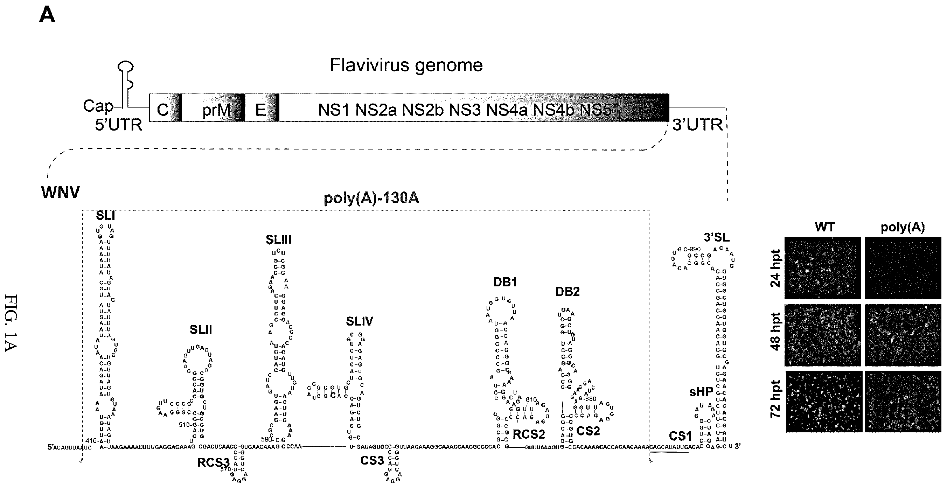

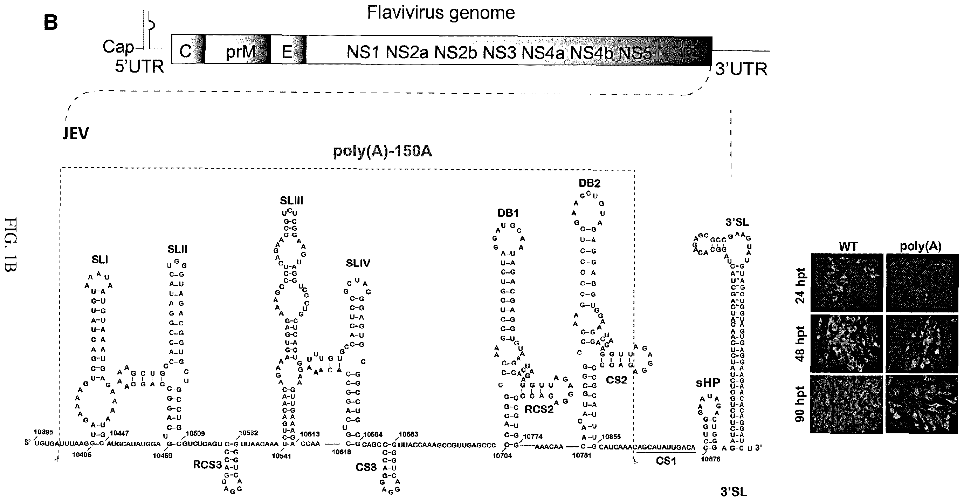

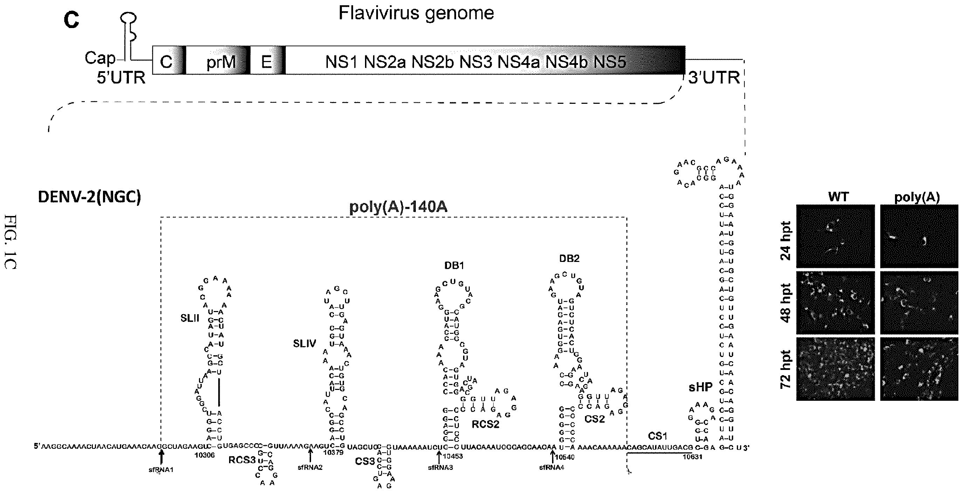

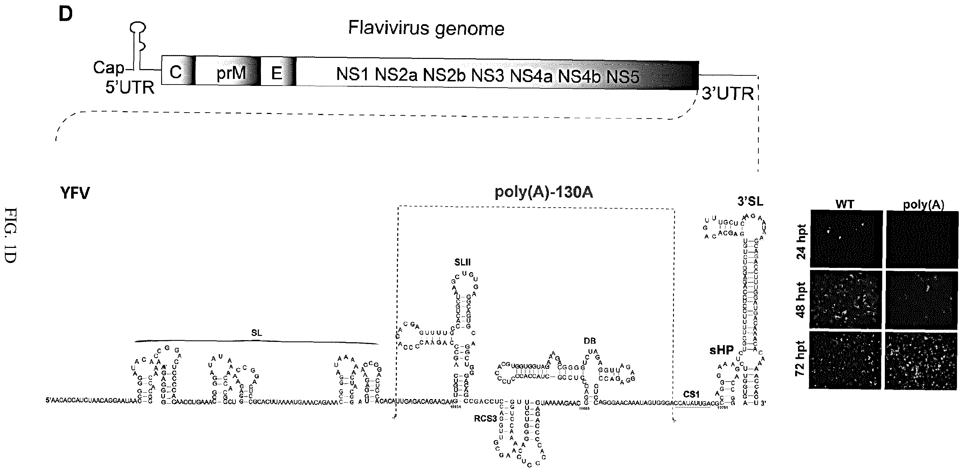

FIG. 1 shows schematic diagrams of clone construction of attenuated flavivirus strains in which 3'UTR is replaced with a poly(A) sequence, and virus rescue, wherein: A: WNV-poly(A) clone construction and virus rescue IFA validation; B: JEV-poly(A) clone construction and virus rescue IFA validation; C: DENV-2(NGC)-poly(A) clone construction and virus rescue IFA validation; and D: YFV-poly(A) clone construction and virus rescue IFA validation.

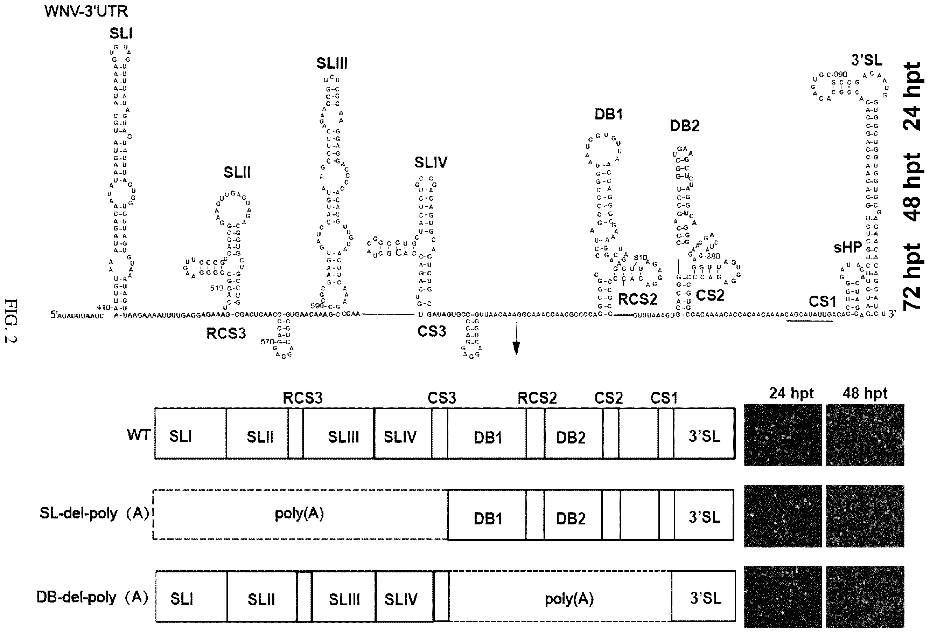

FIG. 2 shows a schematic diagram of clone construction of other attenuated West Nile virus strain, and virus rescue.

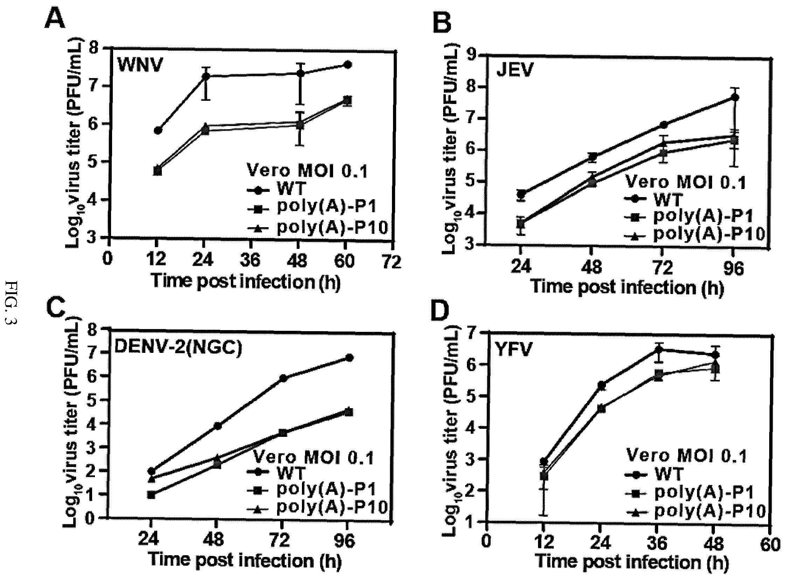

FIG. 3 shows comparisons of a growth curve of an attenuated flavivirus strain in which 3'UTR is replaced with a poly(A) sequence with a growth curve of a wild-type virus, wherein: A: a comparison of a growth curve of a attenuated WNV-poly(A) strain with a growth curve of a corresponding wild-type virus strain; B: a comparison of a growth curve of a JEV-poly(A) strain with the growth curve of the corresponding wild-type virus strain; C: a comparison of a growth curve of a DENV-2(NGC)-poly(A) strain and the growth curve of the corresponding wild-type virus strain; and D: a comparison of a growth curve of a YFV-poly(A) strain with the growth curve of the corresponding wild-type virus strain.

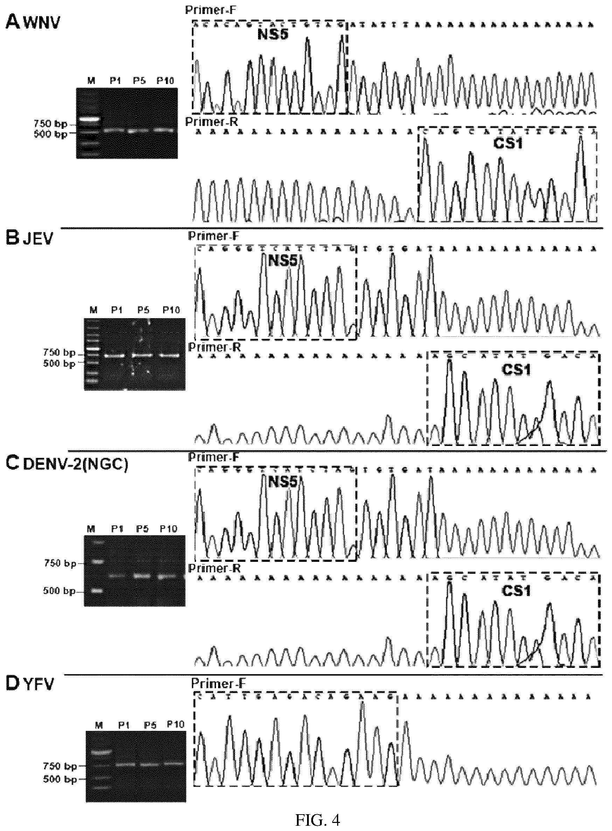

FIG. 4 shows genetic stability verifications of attenuated flavivirus strains in which 3'UTR is replaced with a poly(A) sequence, wherein: A: a genetic stability verification of an attenuated WNV-poly(A) strain; B: a genetic stability verification of an attenuated JEV-poly(A) strain; C: a genetic stability verification of an attenuated DENV-2(NGC)-poly(A) strain; and D: a genetic stability verification of an attenuated YFV-poly(A) strain.

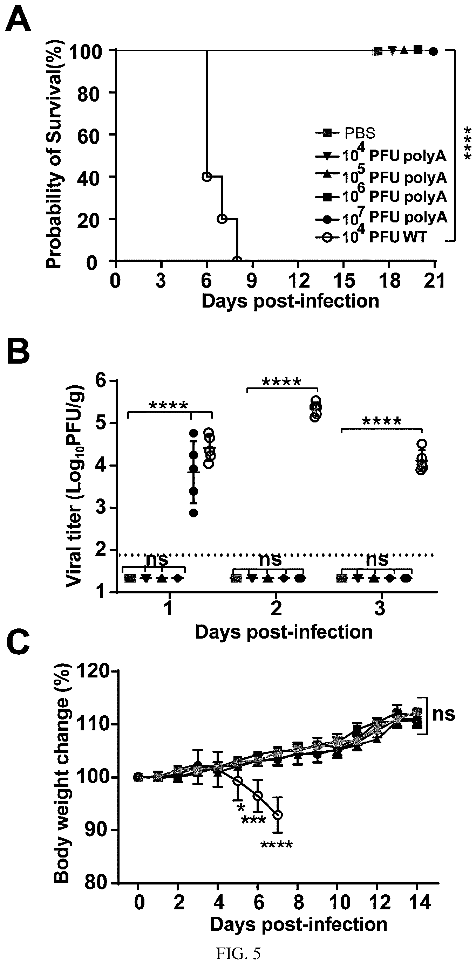

FIG. 5 shows pathogenicity detection of WNV-poly(A) on C57BL/6, wherein: A: a survival rate of mice immunized with an attenuated WNV-poly(A) strain; B: a blood toxicity status of the mice immunized with the attenuated WNV-poly(A) strain; and C: body weights of the mice immunized with the attenuated WNV-poly(A) strain.

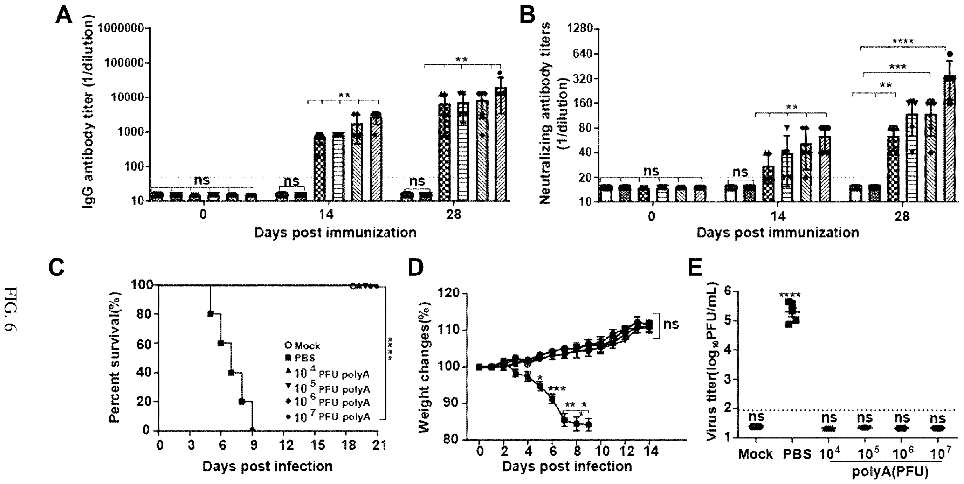

FIG. 6 shows an immunoprotective experiment of WNV-poly(A) on a C57BL/6 mouse model, wherein: A: IgG antibody titers produced by mice immunized singly with an attenuated WNV-poly(A) strain in different dosages; B: neutralizing antibody titers produced by mice immunized singly with the attenuated WNV-poly(A) strain in different dosages; C: survival statuses of mice immunized with singly with the attenuated WNV-poly(A) strain in different dosages after virus challenge; D: body weights of the mice immunized with singly with the attenuated WNV-poly(A) strain in different dosages after virus challenge; and E: blood toxicity statuses of the mice immunized with singly with the attenuated WNV-poly(A) strain in different dosages after virus challenge.

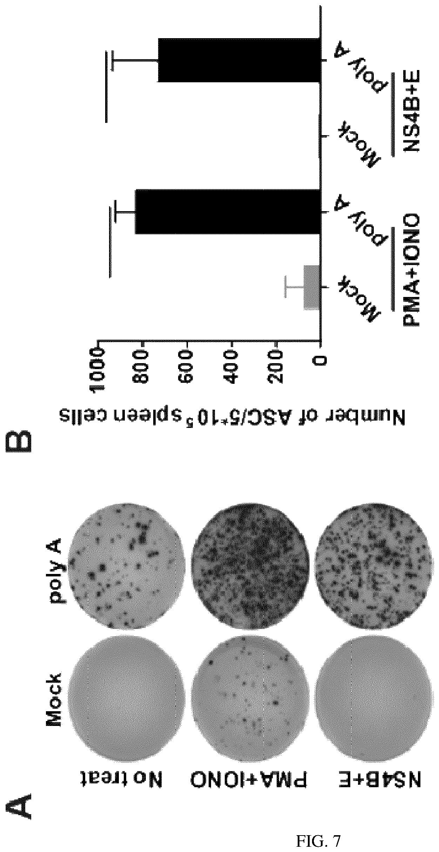

FIG. 7 shows a cellular immunity evaluation of an attenuated WNV-poly(A) strain on a C57BL/6 mouse model, wherein: A: a 7d CD8⁺ IFN-γ⁺ T cell ELISpot detection map of a mouse immunized with singly with the attenuated WNV-poly(A) strain; and B: a 7d CD8⁺ IFN-γ⁺ T cell percentage of the mouse immunized with singly with the attenuated WNV-poly(A) strain.

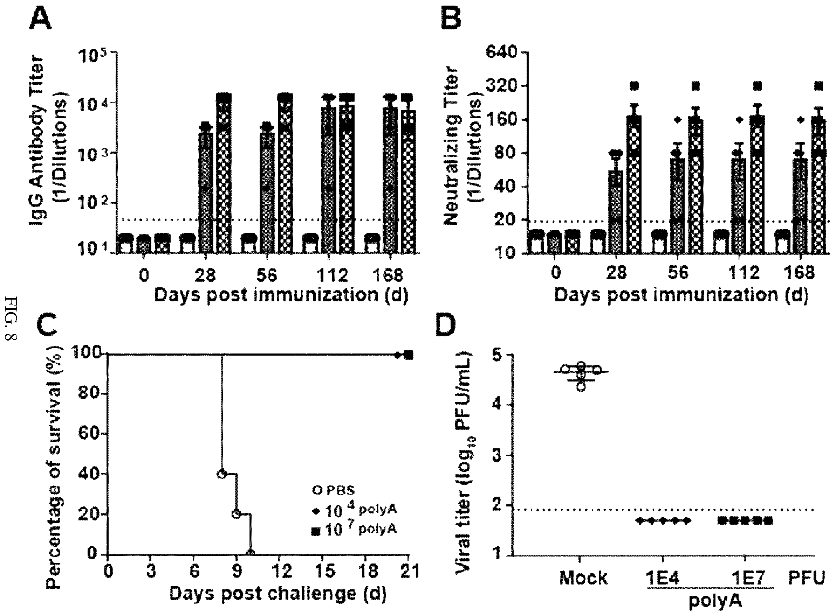

FIG. 8 shows a long-term immune protection experiment of an attenuated WNV-poly(A) strain on a C57BL/6 mouse model, wherein: A: a long-term maintained IgG antibody titer produced by a mouse immunized singly with the attenuated WNV-poly(A) strain; B: a long-term maintained neutralizing antibody production status produced by the mouse immunized singly with the attenuated WNV-poly(A) strain; C: a long-term survival status of the mouse immunized with singly with the attenuated WNV-poly(A) strain after virus challenge; and D: a long-term blood toxicity status of the mouse immunized with singly with the attenuated WNV-poly(A) strain after virus challenge.

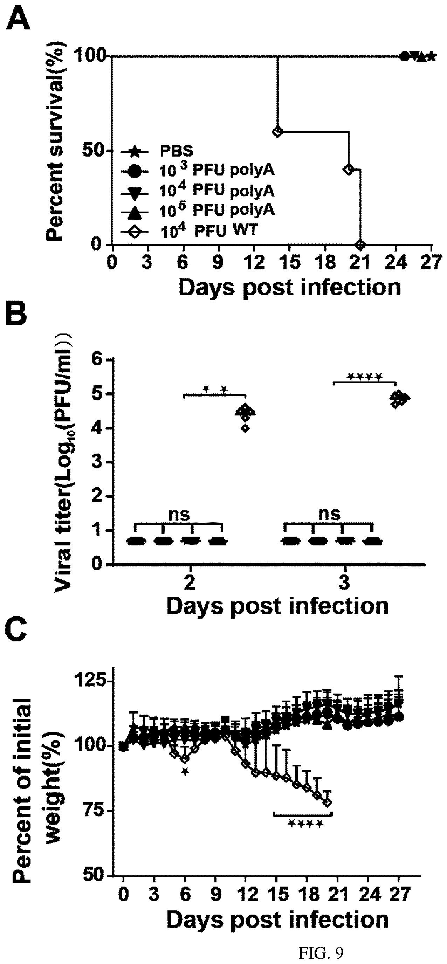

FIG. 9 shows pathogenicity detection of DENV-poly(A) on AG129, wherein: A: a survival rate of mice immunized with an attenuated DENV-poly(A) strain; B: blood toxicity statuses of the mice immunized with the attenuated DENV-poly(A) strain; and C: body weights of the mice immunized with the attenuated DENV-poly(A) strain.

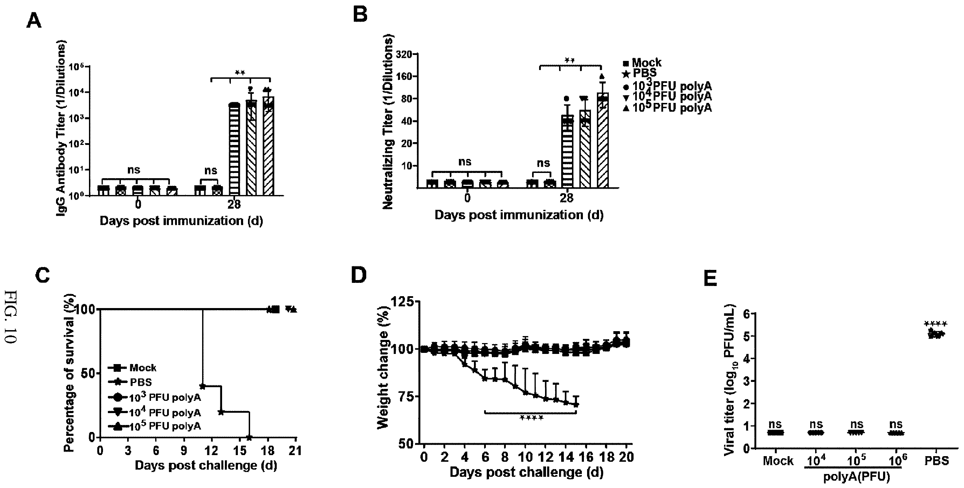

FIG. 10 shows an immune protection experiment of DENV-poly(A) on an AG129/6 mouse model, wherein: A: IgG antibody titers produced by mice immunized singly with an attenuated DENV-poly(A) strain in different dosages; B: neutralizing antibody titers produced by the mice immunized singly with the attenuated DENV-poly(A) strain in different dosages; C: survival statuses of the mice immunized with singly with the attenuated DENV-poly(A) strain in different dosages after virus challenge; D: body weights of the mice immunized with singly with the attenuated DENV-poly(A) strain in different dosages after virus challenge; and E: blood toxicity statuses of the mice immunized with singly with the attenuated DENV-poly(A) strain in different dosages after virus challenge.

### DETAILED DESCRIPTION OF THE INVENTION

### I. Definition

In the present disclosure, unless otherwise defined, all scientific and technical terms as used herein have the meaning as commonly understood by a person skilled in the art. In addition, terms related to proteins, nucleic acid chemistry, molecular biology, cell and tissue culture, microbiology and immunology, and laboratory operating steps as used herein are terms and conventional steps widely used in the respective fields. Meanwhile, in order to better understand the present disclosure, definitions and explanations of related terms are provided below.

As used herein and unless otherwise stated, the term "about" or "approximately" means a range within plus or minus 10% of a given value or range. In cases where an integer is required, this term means an integer within a range plus or minus 10% of a given value or range, or a nearest integer obtained by rounding this range up or down.

As used herein, the flavivirus viruses may include, but are not limited to: Japanese encephalitis virus (JEV), West Nile virus (WNV), tick-borne encephalitis virus (TBEV), yellow fever virus (YFV), dengue virus (DENV), zika virus (ZIKV), St. Louis encephalitis virus (SLEV), Murray Valley encephalitis virus (MVEV), Omsk hemorrhagic fever virus and Kyasanur Forest Disease Virus or any related flavivirus.

As used herein, a 5'-end stem loop region (SL), a dumbbell region (DB), a repetitive cyclization sequence region 3 (RCS3), a cyclization sequence region 3 (CS3), a repetitive cyclization region 2 (RCS2), a cyclization sequence region 2 (CS2), a cyclization sequence region 1 (CS1), and a short hairpin-3' stem loop region (sHP-3'SL) behind the cyclization sequence region 1 in a 3' untranslated region of a flavivirus are the terms in the respective fields. In other studies, the stem loop region (SL) is also referred to as a variable region (VR).

As used herein, the term "live attenuated virus" or "attenuated virus" refers to a virus that has been altered from a primitive parent virus or a wild-type virus in such a manner that its abilities to infect a host, replicate within the host, package, reinfect the host, or combine are attenuated. In general, such attenuation may happen in several or all hosts of the virus, or only in one or more hosts of the virus. Thus, the attenuation of the live attenuated virus (that is, its abilities to infect a host, replicate within the host, package, reinfect the host, or combine are attenuated) is usually associated with one or more hosts of the virus, wherein the live attenuated virus is not significantly or measurably attenuated in one or more other hosts of the virus. The live attenuated virus disclosed herein is usually genetically altered, or may be referred to as being mutated, a mutant, being genetically engineered, a recombinant, or a combination thereof.

As used herein, the term "live attenuated vaccine" or "attenuated vaccine" refers to a pharmaceutical composition comprising a live attenuated pathogen, such as a virus. The pharmaceutical composition comprises at least one immunoactive component, which induces an immune response against the virus in a subject, protects the subject from death or possible death due to the virus, or both, and optionally may include additional components of the immune activity of one or more enhanced active components. A vaccine may additionally include other components typical of the pharmaceutical composition. At least one immunoactive component is one or more live attenuated viruses described herein.

As used herein, the term "virus rescue" refers to a process of introducing in vitro constructs containing virus sequences into appropriate cells to produce invasive or infectious viruses. "Recombinant virus" refers to a genetically engineered virus produced by a recombinant DNA technology, in which a virus sequence is artificially subjected to any genetic manipulations such as deletion, insertion, inversion, and replacement to distinguish this virus from naturally occurring viruses.

As used herein, the term "recombinant RNA virus" refers to the virus described herein comprising a heterologous RNA.

As used herein, the term "wild-type" in the case of viruses refers to a type of virus that is epidemic and naturally propagated and breaks outs as a typical disease. In other embodiments, in the case of viruses, the term "wild type" refers to a parent virus.

As used herein, the term "multiplicity of injection" or "MOI" is an average number of infectious virus particles per infected cell. MOI is calculated by dividing the number of infectious virus particles added (added ml × PFU/ml) by the number of cells added (added ml × cells/ml).

### II. Detailed Description of Embodiments

In one aspect, the present disclosure provides an attenuated flavivirus virus, which comprises a polyadenylic acid (poly(A)) sequence, wherein the polyadenylic acid (poly(A)) sequence is used for replacing a part of nucleotide sequence of a 3' untranslated region (3'UTR) of the flavivirus virus, so that the 3' untranslated region (3'UTR) of the attenuated flavivirus virus obtained in response to the part of the nucleotide sequence of the flavivirus virus being replaced at least retains a 3'-end stem loop region (3'SL).

According to the attenuated flavivirus virus in the previous aspect, the part of nucleotide sequence comprises all or a part of a nucleotide sequence of a 5'-end stem loop region (5' SL), all or a part of a nucleotide sequence of a cyclization sequence region (CS), and/or all or a part of a nucleotide sequence of a dumbbell region (DB) in the 3' untranslated region (3'UTR).

In some embodiments of the present disclosure, after the part of nucleotide sequence is replaced by the polyadenylic acid (poly(A)) sequence, the 3' untranslated region (3'UTR) of the attenuated flavivirus virus retains at least a cyclization sequence region 1 (CS 1) and a short hairpin 3' stem loop region (sHP-3'SL) behind the cyclization sequence region 1 in the 3' untranslated region.

In some preferred embodiments of the present disclosure, the 5'-end stem loop region (5'SL) in the 3' untranslated region (3'UTR) is selected from a stem loop region I (SLI), a stem loop region II (SLII), a stem loop region III (SLIII) and a stem loop region IV (SLIV).

In some preferred embodiments of the present disclosure, the cyclization sequence region (CS) is selected from a cyclization sequence region 1 (CS1), a cyclization sequence region 2 (CS2), a cyclization sequence region 3 (CS3), a repetitive cyclization sequence region 2 (RCS2) and a repetitive cyclization sequence region 3 (RCS3).

In some preferred embodiments of the present disclosure, the dumbbell region (DB) is selected from a dumbbell region 1 (DB 1) and a dumbbell region 2 (DB2).

According to the attenuated flavivirus virus in any of the previous aspects, the polyadenylic acid (poly(A)) sequence comprises 10-200 adenylates.

In some preferred embodiments of the present disclosure, the polyadenylic acid (poly(A)) sequence comprises 50-180, 100-160 adenylates; more preferably, 130-150 adenylates, preferably 130 or 150 adenylates.

In some preferred embodiments of the present disclosure, the flavivirus virus is selected from the group consisting of Japanese encephalitis virus (JEV), West Nile virus (WNV), tick-borne encephalitis virus (TBEV), yellow fever virus (YFV), dengue virus (DENV), zika virus (ZIKV), St. Louis encephalitis virus (SLEV), Murray Valley encephalitis virus (MVEV), Omsk hemorrhagic fever virus and Kyasanur Forest Disease virus.

In some preferred embodiments of the present disclosure, the attenuated flavivirus virus has a plaque titer of 5×10⁴-5×10⁶ PFU/ml.

In some preferred embodiments of the present disclosure, the attenuated flavivirus virus is 10-100 times less virulent than a parent wild-type virus.

In some preferred embodiments of the present disclosure, the attenuated flavivirus virus is obtained by means of rescue on baby hamster kidney cells BHK-21 and/or amplification on African green monkey kidney Vero cells.

In some preferred embodiments of the present disclosure, the dengue virus is selected from 1-type, 2-type, 3-type and 4-type dengue viruses.

In some preferred embodiments of the present disclosure, the attenuated flavivirus virus is an attenuated West Nile virus. In some preferred embodiments of the present disclosure, the attenuated West Nile virus comprises a nucleotide sequence having 80% or more identity with a nucleotide sequence shown in SEQ ID NO. 1, preferably a nucleotide sequence having more than 85%, 90%, 95%, 96%, 97%, 98%, 99% identity with the nucleotide sequence shown in SEQ ID NO. 1, more preferably a nucleotide sequence having 98% or 99% or more identity with the nucleotide sequence shown in SEQ ID NO. 1. In a specific embodiment of the present disclosure, the nucleotide sequence of the attenuated West Nile virus is shown in SEQ ID NO. 1.

In some preferred embodiments of the present disclosure, the attenuated flavivirus virus is an attenuated Japanese encephalitis virus. In some preferred embodiments of the present disclosure, the attenuated Japanese encephalitis virus comprises a nucleotide sequence having 80% or more identity with a nucleotide sequence shown in SEQ ID NO. 2, preferably a nucleotide sequence having more than 85%, 90%, 95%, 96%, 97%, 98%, 99% identity with the nucleotide sequence shown in SEQ ID NO. 2, more preferably a nucleotide sequence having 98% or 99% or more identity with the nucleotide sequence shown in SEQ ID NO. 2; more preferably, the nucleotide sequence of the attenuated Japanese encephalitis virus is SEQ ID NO. 2. In a specific embodiment of the present disclosure, the nucleotide sequence of the attenuated Japanese encephalitis virus is shown in SEQ ID NO. 2.

In some preferred embodiments of the present disclosure, the attenuated flavivirus virus is an attenuated dengue virus. In some preferred embodiments of the present disclosure, the attenuated dengue virus comprises a nucleotide sequence having 80% or more identity with a nucleotide sequence shown in SEQ ID NO. 3, preferably a nucleotide sequence having more than 85%, 90%, 95%, 96%, 97%, 98%, 99% identity with the nucleotide sequence shown in SEQ ID NO. 3, more preferably a nucleotide sequence having 98% or 99% or more identity with the nucleotide sequence shown in SEQ ID NO. 3; more preferably, the nucleotide sequence of the attenuated dengue virus is SEQ ID NO. 3. In a specific embodiment of the present disclosure, the nucleotide sequence of the attenuated dengue virus is shown in SEQ ID NO. 3.

In some preferred embodiments of the present disclosure, the attenuated flavivirus virus is an attenuated yellow fever virus. In some preferred embodiment of the present disclosure, the attenuated yellow fever virus comprises a nucleotide sequence having 80% or more identity with a nucleotide sequence shown in SEQ ID NO. 4, preferably a nucleotide sequence having more than 85%, 90%, 95%, 96%, 97%, 98%, 99% identity with the nucleotide sequence shown in SEQ ID NO. 4, more preferably a nucleotide sequence having 98% or 99% or more identity with the nucleotide sequence shown in SEQ ID NO. 4; more preferably, the nucleotide sequence of the attenuated yellow fever virus is SEQ ID NO. 4. In a specific embodiment of the present disclosure, the nucleotide sequence of the attenuated yellow fever virus is shown in SEQ ID NO. 4.

In some preferred embodiments of the present disclosure, the attenuated flavivirus virus is an attenuated zika virus. In some preferred embodiments of the present disclosure, the attenuated zika virus comprises a nucleotide sequence having 80% or more identity with a nucleotide sequence shown in SEQ ID NO. 5, preferably a nucleotide sequence having more than 85%, 90%, 95%, 96%, 97%, 98%, 99% identity with the nucleotide sequence shown in SEQ ID NO. 5, more preferably a nucleotide sequence having 98% or 99% or more identity with the nucleotide sequence shown in SEQ ID NO. 5; more preferably, the nucleotide sequence of the attenuated zika virus is SEQ ID NO. 5. In a specific embodiment of the present disclosure, the nucleotide sequence of the attenuated zika virus is shown in SEQ ID NO. 5.

In some preferred embodiments of the present disclosure, the attenuated flavivirus virus is an attenuated tick-borne encephalitis virus. In some preferred embodiments of the present disclosure, the attenuated tick-borne encephalitis virus comprises a nucleotide sequence having 80% or more identity with a nucleotide sequence shown in ID NO. 6, preferably a nucleotide sequence having more than 85%, 90%, 95%, 96%, 97%, 98%, 99% identity with the nucleotide sequence shown in SEQ ID NO. 6, more preferably a nucleotide sequence having 98% or 99% or more identity with the nucleotide sequence shown in SEQ ID NO. 6; more preferably, the nucleotide sequence of the attenuated tick-borne encephalitis virus is SEQ ID NO. 6. In a specific embodiment of the present disclosure, the nucleotide sequence of the attenuated tick-borne encephalitis virus is shown in SEQ ID NO. 6.

In one preferred embodiment of the present disclosure, the part of nucleotide sequence comprises a 5'-end stem loop region I (SLI), a stem loop region II (SLII), a repetitive cyclization sequence region 3 (RCS3), a stem loop region III (SLIII), a stem loop region IV (SLIV), and a cyclization sequence region 3 (CS3), and after the part of nucleotide sequence is replaced by the polyadenylic acid (poly(A)) sequence, the 3' untranslated region (3'UTR) of the attenuated flavivirus virus retains a dumbbell region 1 (DB1), a repetitive cyclization sequence region 2 (RCS2), a dumbbell region 2 (DB2), a cyclization sequence region 2 (CS2), a cyclization sequence region 1 (CS1) and a short hairpin-3' stem loop region (sHP-3'SL) behind the cyclization sequence region 1 in the 3' untranslated region.

In one preferred embodiment of the present disclosure, the part of nucleotide sequence comprises a dumbbell region 1 (DB 1), a repetitive cyclization sequence region 2 (RCS2), a dumbbell region 2 (DB2), a cyclization sequence region 2 (CS2), and a cyclization sequence region 1 (CS1), and after the part of nucleotide sequence is replaced by the polyadenylic acid (poly(A)) sequence, the 3' untranslated region (3'UTR) of the attenuated flavivirus virus retains a 5'-end stem loop region I (SLI), a stem loop region II (SLII), a repetitive cyclization sequence region 3 (RCS3), a stem loop region III (SLIII), a stem loop region IV (SLIV), a cyclization sequence region 3 (CS3), and a short hairpin-3' stem loop region (sHP-3'SL) in the 3' untranslated region.

In one preferred embodiment of the present disclosure, the part of nucleotide sequence comprises a 5'-end stem loop region I (SLI), a stem loop region II (SLII), a repetitive cyclization sequence region 3 (RCS3), a stem loop region III (SLIII), a stem loop region IV (SLIV), a cyclization sequence region 3 (CS3), a dumbbell region 1 (DB 1), a repetitive cyclization sequence region 2 (RCS2), a dumbbell region 2 (DB2), and a cyclization sequence region 2 (CS2), and after the part of nucleotide sequence is replaced by the polyadenylic acid (poly(A)) sequence, the 3' untranslated region (3'UTR) of the attenuated flavivirus virus retains a cyclization sequence region 1 (CS1) and a short hairpin-3' stem loop region (sHP-3'SL) behind the cyclization sequence region 1 in the 3' untranslated region.

In one preferred embodiment of the present disclosure, the part of nucleotide sequence comprises a 5'-end stem loop region II (SLII), a repetitive cyclization sequence region 3 (RCS3), a stem loop region IV (SLIV), a cyclization sequence region 3 (CS3), a dumbbell region 1 (DB 1), a repetitive cyclization sequence region 2 (RCS2), a dumbbell region 2 (DB2), and a cyclization sequence region 2 (CS2), and after the part of nucleotide sequence is replaced by the poly(A)) sequence, the 3' untranslated region (3'UTR) of the attenuated flavivirus virus retains a cyclization sequence region 1 (CS1) and a short hairpin 3' stem loop region (sHP-3'SL) behind the cyclization sequence region 1 in the 3' untranslated region.

In one preferred embodiment of the present disclosure, the part of nucleotide sequence comprises a 5'-end stem loop region I (SLII), a repetitive cyclization sequence region 3 (RCS3), a dumbbell region (DB), a repetitive cyclization sequence region 2 (RCS2), a dumbbell region 2 (DB2), and a cyclization sequence region 2 (CS2), and after the part of nucleotide sequence is replaced by the polyadenylic acid (poly(A)) sequence, the 3' untranslated region (3'UTR) of the attenuated flavivirus virus retains 5'-end stem loop region (SL), a cyclization sequence region 1 (CS1) and a short hairpin-3' stem loop region (sHP-3'SL) behind the cyclization sequence region 1 in the 3' untranslated region.

In an aspect, the present disclosure provides a DNA, which can be transcribed to produce an RNA genome of the attenuated flavivirus virus.

In some embodiments of the present disclosure, the DNA is an infectious clone of an attenuated strain of the flavivirus virus.

In some preferred embodiments of the present disclosure, the infectious clone is a plasmid. In an aspect, the present disclosure provides a cell, which comprises the attenuated flavivirus virus or the DNA.

In an aspect, the present disclosure provides a vaccine, which comprises the attenuated flavivirus virus, the DNA and/or the cell.

In an aspect, the present disclosure provides a pharmaceutical composition, which comprises the attenuated flavivirus virus, the DNA, the cell and/or the vaccine, and a pharmaceutically acceptable vector.

In an aspect, the present disclosure provides a method for preparing the attenuated flavivirus virus, the method comprising substituting all or a part of nucleotide sequence of a 3' untranslated region (3'UTR) of a wild-type virus with a polyadenylic acid (poly(A)) sequence.

In some preferred embodiments of the present disclosure, the attenuated flavivirus virus is obtained by means of rescue on baby hamster kidney cells BHK-21 and/or amplification on African green monkey kidney Vero cells.

In an aspect, the present disclosure provides use of the attenuated flavivirus virus, the DNA, and the cell in the preparation of an attenuated flavivirus virus vaccine.

In one preferred embodiment of the present disclosure, the use is use of the attenuated West Nile virus in the preparation of an attenuated West Nile virus vaccine.

In one preferred embodiment of the present disclosure, the use is use of the attenuated Japanese encephalitis virus in the preparation of an attenuated Japanese encephalitis virus vaccine.

In one preferred embodiment of the present disclosure, the use is use of the attenuated dengue virus in the preparation of an attenuated dengue virus vaccine.

In one preferred embodiment of the present disclosure, the use is use of the attenuated yellow fever virus in the preparation of an attenuated yellow fever virus vaccine.

In one preferred embodiment of the present disclosure, the use is use of the attenuated zika virus in the preparation of an attenuated zika virus vaccine.

In an aspect, the present disclosure provides use of the attenuated flavivirus virus, the DNA, the cell, the vaccine and/or the pharmaceutical composition in the preparation of a medicament for treating or preventing diseases caused by a flavivirus virus.

In one preferred embodiment of the present disclosure, the use is use of the attenuated West Nile virus in the preparation of a medicament for treating or preventing diseases caused by the West Nile virus.

In one preferred embodiment of the present disclosure, the use is use of the attenuated Japanese encephalitis virus in the preparation of a medicament for treating or preventing diseases caused by the Japanese encephalitis virus.

In one preferred embodiment of the present disclosure, the use is use of the attenuated dengue virus in the preparation of a medicament for treating or preventing diseases caused by the dengue virus.

In one preferred embodiment of the present disclosure, the use is use of the attenuated yellow fever virus in the preparation of a medicament for treating or preventing diseases caused by the yellow fever virus.

In one preferred embodiment of the present disclosure, the use is use of the attenuated zika virus in the preparation of a medicament for treating or preventing diseases caused by the zika virus.

In one aspect, the present disclosure provides a method for stimulating an immune response to a flavivirus virus, the method comprising immunological administration of the attenuated flavivirus virus, the DNA, the cell, the vaccine and/or the pharmaceutical composition.

An embodiment herein provides administration of a composition to a subject in a biocompatible form suitable for in vivo administration. "Biocompatible form suitable for in vivo administration" refers to a form of an active agent to be administered (e.g., a medicament in an embodiment), wherein a therapeutic effect of the active agent exceeds any toxic effect. The administration of a therapeutic composition in a therapeutically effective amount is defined as an effective amount in the dose and time period necessary to achieve a desired result. For example, the therapeutically active amount of a compound can vary with factors such as an individual's disease status, age, sex, and weight, as well as the ability of an antibody to elicit a desired response in an individual. A dosage regimen can be adjusted to provide an optimal response to treatment.

In one embodiment, a compound (e.g., a live attenuated virus in an embodiment) may be administered conveniently, e.g., by subcutaneous administration, intravenous administration, oral administration, inhalation, intradermal application, transdermal application, intravaginal application, topical administration, intranasal administration or rectal application. Depending on the route of administration, the active compound may be included in a protective buffer (e.g., albumin and trehalose, poloxamer 407/trehalose/albumin (FTA)). In one embodiment, the composition may be administered orally. In another embodiment, the composition may be administered intravenously. In one embodiment, the composition may be administered intranasally, for example, inhalation. In another embodiment, the composition may be administered intradermally using a needle-free system (e.g., Pharmajet^{®}) or other intradermal application systems.

In certain embodiments, live attenuated virus vaccines (e.g., a West Nile virus vaccine) may be administered to children or young adults in the form of stable formulations (e.g., albumin and trehalose; FTA, or other formulations used to stabilize the live attenuated viruses). As used herein, a "pharmaceutically acceptable vector" may also include diluents, such as saline and an aqueous buffer solution. It may be necessary to combine a live attenuated virus formulation with a material that prevents its inactivation, or to apply the compound together with a material that prevents its inactivation. In certain embodiments, one or more formulations of a live attenuated West Nile virus may be administered subcutaneously or intradermally to a subject in an initial dose followed by a booster dose. Dispersants may also be prepared in glycerol, liquid polyethylene glycol and a mixture thereof, and in oil. Under normal storage and use conditions, these formulations may contain preservatives to prevent microbial growth or other stabilization formulations (e.g., for stabilizing live attenuated flaviviruses or other stable formulations).

Pharmaceutical compositions suitable for injectable use may be administered by means known in the art. For example, sterile aqueous solutions (in the case of water solubility) or dispersions and sterile powders for the immediate preparation of sterile injectable solutions or dispersions can be used. In some embodiments, the composition may be sterile and may be a fluid with ease of injectability. Pharmaceutically acceptable vectors may be solvents or dispersion media containing, for example, water, ethanol, polyols (e.g., glycerol, propylene glycol and liquid polyethylene glycol) and suitable mixtures thereof. For example, appropriate flowability may be maintained by using a coating material such as lecithin, by maintaining the desired particle size in the case of dispersions, and by using surfactants. Prevention of microorganisms can be achieved by adding various antibacterial agents or antifungal agents or other reagents.

In some embodiments, when formulated, a solution may be administered in compatiblity with a dose formulation and administrated in a therapeutically effective amount. According to these embodiments, the formulation may be easily administered in a variety of dosage forms, such as the type of the injectable solution described above.

In certain embodiments, a single dose or multiple doses of attenuated flavivirus (e.g., West Nile virus) formulation may also be administered to a subject. In some embodiments, the subject may be treated with a single dose of formulation. In other embodiments, the subject may be treated with at least two doses of live attenuated West Nile virus formulations. In certain embodiments, the subject may be administered with an attenuated West Nile virus composition on day 0, and with a booster dose within about 3 months of the first dose. In certain embodiments, the subject is an unimmunized subject who has never been exposed to a West Nile virus (seronegative). In other embodiments, the subject may have been previously exposed to the West Nile virus and/or West Nile virus infection (seropositive). According to these embodiments, the seronegative subject may be treated on Day 0 and then accept a booster dose within 6 months, 5 months, 4 months, 3 months or less of the first dose in order to produce an enhanced immune response to the West Nile virus. In certain embodiments, the child may be a child from about 2 years old to about 17 years old. In other embodiments, the child may be 2 to 17 years old.

In another embodiment, a nasal solution or spray, aerosol or inhaler may be used to deliver a live attenuated flavivirus (e.g., a West Nile virus) virus formulation to the subject. Some formulations may contain excipients, e.g., pharmaceutical grade mannitol, lactose, starch, magnesium stearate, sodium saccharin, cellulose, and magnesium carbonate.

A pharmaceutical composition may be prepared from a vector that protects active ingredients from rapid elimination from the body, e.g., releases a formulation or coating material on time. Such vectors may include controlled-release formulations such as, but not limited to, delivery systems for microencapsulation and biodegradable biocompatible polymers such as vinyl ethyleneacetate, polyanhydride, polyglycolic acid, polyorthoesters, polylactic acid and other known substances.

In certain embodiments, the dose range of the live attenuated flavivirus (e.g., West Nile virus) may be about 10² to about 10⁶ PFU initially administered and optionally, followed by at least a second administration within 30 days or up to 12 months thereafter as needed. In certain embodiments, the pharmaceutical composition disclosed herein may be administered to the subject in one or more doses. In some embodiments, an immunogenic composition disclosed herein may be administered to the subject in a single dose or two or more within a predetermined period of time, including, but not limited to, within about 6 months, within about 120 days, within about 90 days, within about 80 days, within about 70 days, within about 60 days, within about 50 days, about 40 days, about 30 days, about 20 days, about 10 days, about 5 days or less, or administered to the subject within hours or minutes on the same day or simultaneously administered in the same or different anatomical locations. In some embodiments, the pharmaceutical compositions disclosed herein may be administered within about 90 days of each other, within about 60 days of each other, within about 30 days of each other, and within about less than 30 days of each other. In some embodiments, the composition disclosed herein may be administered to a subject subcutaneously or intradermally. Applications in two or more anatomical sites may include any combination of applications, including by the same pattern in two or more anatomical sites or by two different modes of separate anatomical sites. According to these embodiments, two or more anatomical sites may include different limbs or different areas of the body. In certain embodiments, two doses of vaccine composition may be continuously introduced into the subject on Day 0 in the same or multiple anatomical locations, e.g., to provide protection against all flavivirus (e.g., West Nile virus) serotypes (e.g., cross-protection). In other embodiments, the pharmaceutical composition may include a combination of a live attenuated West Nile virus with other other immunogenic reagents against other flaviviruses (e.g., Zika virus, Japanese encephalitis virus, West Nile virus, St. Louis encephalitis virus, yellow fever virus, or other viruses). In certain embodiments, a vaccine against the West Nile virus disclosed herein may be used to reduce other related virus infections, such as Zika virus infection.

In some embodiments, the pharmaceutical composition disclosed herein may be used to increase an immune response of a target formulation in the subject, which is performed by combining immunogenic compositions against the flavivirus (e.g., West Nile virus) virus in children or young adults administrated with such pharmaceutical compositions against the flavivirus (e.g., West Nile virus) virus with reagents that enhance CD8⁺T cell responses or other immune responses against the West Nile virus.

Topical application is accomplished by topical application of creams, gels, rinses, etc. containing therapeutically effective amounts of serine protease inhibitor. Transdermal administration is accomplished through the application of creams, douches, gels, etc. that enable the serine protease inhibitor to penetrate the skin and enter the bloodstream. In addition, a permeation pump may be used for application. The necessary dose will vary depending on specific conditions being treated, a method of administration, and a rate at which molecules are cleared from the body.

Other embodiments relate to methods for reducing the inactivation of live attenuated viruses, including but not limited to combining one or more live attenuated viruses with compositions capable of reducing the inactivation of the live attenuated viruses (e.g., flavivirus). These compositions may include, but are not limited to, one or more protein reagents; one or more sugar or polyol reagents; and optionally one or more EO-PO block copolymers, wherein the compositions may reduce the inactivation of the activated attenuated viruses or stabilize the activated attenuated viruses.

In certain embodiments, the compositions covered herein may be partially or completely dehydrated or hydrated. In other embodiments, a stable protein reagent comprising the pharmaceutical or non-pharmaceutical composition used herein may include, but is not limited to, whey protein, human serum albumin, recombinant human serum albumin (rHSA), bovine serum albumin (BSA), other serum albumin or albumin gene family members. Sugar or polyol reagents may include, but are not limited to, monosaccharides, disaccharides, sugar alcohols, trehalose, sucrose, maltose, isomaltose, cellobiose, gentiobiose, laminaribose, xylobiose, mannobiose, lactose, fructose, sorbitol, mannitol, lactitol, xylitol, erythritol, raffinose, amylase, cyclodextrin, chitosan, or cellulose. In certain embodiments, surfactants may include, but are not limited to, nonionic surfactants, such as an alkyl poly(ethylene oxide), poly(ethylene oxide) and poly(propylene oxide) copolymer (EO-PO block copolymer), poly(vinyl pyrrolidone), alkyl polyglycoside (e.g., sucrose monostearate, lauryl diglucoside or sorbitan monolaureate, capryl glucoside and decyl maltoside), fatty alcohol (cetyl alcohol or olelyl alcohol or cocamides (cocamide MEA, cocamide DEA and cocamide TEA).

In other embodiments, surfactants may include, but are not limited to, poloxamer 407 (e.g., Pluronic F127^{®}), alternative poloxamer 407 or poloxamer 335, 338 or 238 other than poloxamer 407, or other EO-PO block copolymers having similar properties to F127^{®}.

In some embodiments, a vaccine composition may include, but is not limited to, one or more protein reagents, which are serum albumin; one or more trehalose sugars; and one or more surfactant polymer reagents, such as an EO-PO block copolymer, poloxamer 407 or, more specifically, Pluronic F127^{®}.

In other embodiments, formulations for stabilizing live viruses may comprise one or more live flaviviruses, one or more carbohydrate reagents and one or more amino acids or salts, esters or amide derivatives thereof. In other embodiments, the formulations used herein stabilizes the activated attenuated flavivirus for commercial use. In some embodiments, the composition further comprises a buffer. According to these embodiments, the buffer may include, but are not limited to, phosphate-buffered saline (PBS). According to these embodiments, the buffer may include at least one of sodium chloride (NaCl), monosodium phosphate and/or disodium phosphate (Na₂HPO₄), potassium chloride (KCl) and potassium phosphate (KH₂PO₄). In some embodiments, the buffer of the composition may comprise sodium chloride having a concentration of 25 mM to 200 mM. In other embodiments, the composition disclosed herein may comprise other suitable reagents, such as urea and/or MSG.

In some embodiments, live attenuated flaviviruses such as a West Nile virus may be stabilized in formulations, the formulations comprising but not limited to: recombinant HSA having a concentration of 0.1% to 0.2% (w/v); and/or sucrose having a concentration of about 4.0% to about 6.0% (w/v); and/or mannitol having a concentration of about 2% to 4% (w/v); and/or alanine having a concentration of about 8.0 mM to about 22.0 mM; and/or methionine having a concentration of about 1.0 mM to about 5.0 mM; and/or MSG having a concentration of about 8.0 mM to 12.0 mM; and/or urea having a concentration of about 0.1% to about 0.3% (w/v). In certain embodiments, the composition may comprise recombinant HSA, trehalose, mannitol, alanine, methionine, MSG and urea. In other embodiments, the stabilization composition may comprise HSA having a concentration of about 0.1% to about 0.2% (w/v); trehalose having a concentration of about 4% to about 6% (w/v); mannitol having a concentration of about 2% to about 4% (w/v); alanine having a concentration of 8 mM to 22 mM; methionine having a concentration of 1 mM to 5 mM; MSG having a concentration of 8mM to 12mM; urea having a concentration of 0.1% to 0.3% (w/v). Certain formulations for stabilizing live attenuated viruses may include, but are not limited to, recombinant HSA, sucrose, alanine, and urea. According to these embodiments, the HSA concentration may be about 0.1% to about 0.2% (w/v); the sucrose concentration may be about 4% to about 6% (w/v); the alanine concentration may be about 8.0 mM to about 22 mM, and the urea concentration may be about 0.1% to about 0.3% (w/v). Other stabilization formulations may include recombinant HSA, sucrose, methionine, and urea. The recombinant HSA concentration may be about 0.1% to 0.2% (w/v); the sucrose concentration may be about 4.0% to about 6.0% (w/v); the methionine concentration may be about 1.0 mM to about 5.0 mM, and the urea concentration may be about 0.1% to about 0.3% (w/v). In other embodiments, the stabilization formulation may comprise recombinant HSA, sucrose, arginine, and urea, wherein the recombinant HSA concentration may be 0.1% to 0.2% (w/v); the sucrose concentration may be 4% to 6% (w/v); the arginine concentration may be 10 mM to 50 mM; and the urea concentration may be 0.1% to 0.3% (w/v). Other stabilization formulations may include recombinant HSA, trehalose, arginine, and urea, wherein the recombinant HSA concentration is about 0.1% to 0.2% (w/v); the trehalose concentration is about 4% to 6% (w/v); the arginine concentration is about 10 mM to 50 mM; and the urea concentration is about 0.1% to 0.3% (w/v). In other embodiments, the stabilization composition may include recombinant HSA, trehalose, MSG and urea. According to these embodiments, the HSA concentration may be about 0.1% to about 0.2% (w/v); the trehalose concentration may be about 4.0% to about 6.0% (w/v); the MSG concentration may be 8.0 mM to 22 mM, and the urea concentration may be 0.1% to 0.3% (w/v).

Some embodiments herein relate to a live attenuated virus composition for partial or complete dehydration caused by transport or other reasons. According to these embodiments, the composition may be dehydrated by 20% or more; 30% or more; 40% or more; 50% or more; 60% or more; 70% or more; 80% or more; or 90% or more. According to these embodiments, prior to administering a pharmaceutically acceptable composition to a subject, a virus vaccine composition may be dehydrated and rehydrated in any known stabilization composition.

In certain embodiments, the subject may be a mammal, such as a human or a veterinarian and/or a domesticated animal or a domestic animal or wild animal. In certain embodiments, the immunogenic composition disclosed herein may be effective in immunizing juvenile subjects, such as human children aged 20 years or younger. Although the prior art related to current dengue virus vaccines/immunogenic compositions (e.g., dengue/yellow fever chimera) used in children has reported low efficacy and/or immunogenicity in children aged 9 years or younger, immunogenic compositions disclosed herein can produce effective immune responses in children aged about 1 to about 17 years or about 1 to about 9 years or older. Compared with the prior art, the immunogenic composition disclosed herein exhibits excellent efficacy and immunogenicity.

In certain embodiments, immunogenic compositions against flaviviruses (e.g., West Nile virus) may include one or more of the followings: an attenuated West Nile virus having a concentration of about 1.0×10³ to about 5×10⁵ PFU; a live West Nile virus having a concentration of about 1.0×10³ to about 5×10⁵ PFU; an attenuated West Nile virus having a concentration of about 5.0×10³ to about 5×10⁵ PFU; and/or an attenuated West Nile virus having a concentration of about 1.0x10⁴ to about 5×10⁶ PFU. In some embodiments, the subject treated by the composition and method disclosed herein may be treated 2 times a year, annually, 18 months, or a similar regimen, depending on, for example, a location and travel plan of the subject. In certain embodiments, immunogenic compositions against the West Nile virus may include one or more of the followings: an attenuated West Nile virus having a concentration of about 2.0×10⁴ PFU; a live West Nile virus having a concentration of about 5.0×10³ PFU; an attenuated West Nile virus having a concentration of about 1×10⁵ PFU; and/or an attenuated West Nile virus having a concentration of about 3.0×10⁵ PFU.

Compared with the prior art, the present invention has the following advantages and effects.
1. A reverse genetics technology used in the present disclosure to construct attenuated flavivirus viruses (such as an attenuated West Nile virus strain WNV-poly(A)) has the advantages of advanced maturity, convenience, simplicity, and controllable positioning.
2. The attenuated flavivirus virus (e.g., WNV-poly(A)) provided in the present disclosure can be cultivated and amplified in Vero to obtain a high-titer virus, which is simple to obtain, thereby facilitating the production of subsequent vaccines and saving the cost.
3. The attenuated flavivirus virus (e.g., WNV-poly(A)) provided in the present disclosure maintains the sequence deletion of SL and DB regions and the stability of a -poly(A) sequence during continuous passage of 3'UTR, thereby achieving good genetic stability.
4. The attenuated flavivirus virus (e.g., WNV-poly(A)) provided by the present disclosure which infects C57BL/6 mice at high dose does not cause any illness, has been shown to be sufficiently attenuated in mice, and is thus high in security. A single immunization of 10⁴-10⁷ PFU of WNV-poly(A) can enable the mice to produce high levels of WNV-specific IgG antibodies and neutralizing antibodies, which can provide complete immune protection for the mice after being challenged at a high lethal dose of WT WNV.
5. The attenuated flavivirus virus (e.g., WNV-poly(A)) provided by the present disclosure is immunized at low dose (10⁴ PFU) or high dose (10⁷ PFU) in a C57BL/6 mouse model to maintain a high humoral immune response for a long time, the level of neutralizing antibodies remains stable, and complete immune protection is still provided to the mice after being challenged at a high lethal dose of WT WNV

The above results show that the general preparation method of the attenuated flavivirus strain provided in the present disclosure can be used to prepare safe and effective attenuated vaccine strains, which have good application prospects and important strategic significance for China and the world to deal with the potential threat of flavivirus.

The experimental methods of plasmid linearization, RNA in vitro transcription, transfection, viral titer determination, etc. mentioned in this section are routinely used in the art unless otherwise specified. The followings are only several specific embodiments of the present disclosure. Obviously, the present disclosure is not limited to the following embodiments, and may have many variations. Therefore, modifications or improvements made by those skilled in the art on the basis of the present disclosure should fall within the protection scope of the present disclosure.

### Example 1: Construction of attenuated flavivirus strain clone and virus rescue 1.Construction of attenuated flavivirus strain

A schematic diagram of the construction of an attenuated flavivirus strain is shown in FIG. 1.

Using a plasmid DNA from an infectious clone of a wild-type (WT) West Nile virus (WNV) strain as a framework, an attenuated strain plasma of the WNV-poly (A) virus is obtained by deleting sequences from SLI to CS2 in a 3' untranslated region (3'UTR) of WNV (including a 5'-end stem loop region I (SLI), a stem loop region II (SLII), a repetitive cyclization region 3 (RCS3), a stem loop region III (SLIII), a stem loop region IV (SLIV), a cyclization sequence region 3 (CS3), a dumbbell region 1 (DB 1), a repetitive cyclization region 2 (RCS2), a dumbbell region (DB2), and a cyclization sequence region 2 (CS2)), retaining the cyclization sequence region 1 (CS1) and a short hairpin-3' stem loop region (sHP-3'SL) behind the cyclization sequence region 1 in the 3' untranslated region, and inserting a poly(A) sequence (130 adenylates) to a deletion site resulting from deletion of the sequences from SLI to CS2, and a nucleotide sequence of the attenuated strain plasma is shown in SEQ ID NO. 1.

Using a plasmid DNA from an infectious clone of a wild-type (WT) Japanese encephalitis virus strain (JEV) as a framework, an attenuated strain plasma of the JEV-poly (A) virus is obtained by deleting sequences from SLI to CS2 in a 3' untranslated region (3'UTR) of JEW( including SLI, SLII, RCS3, SLIII, SLIV, CS3, DB1, RCS2, DB2 and CS2), retaining a cyclization sequence region 1 (CS1) and a short hairpin-3' stem loop region (sHP-3'SL) behind the cyclization sequence region 1 in the 3' untranslated region, and inserting a poly(A) sequence (150 adenylates) to a deletion site resulting from deletion of the sequences from SLI to CS2, and a nucleotide sequence of the attenuated strain plasma is shown in SEQ ID NO. 2.

Using a plasmid DNA from an infectious clone of a wild-type (WT) dengue virus type-2 New Guinea Island strain (DENV-2 (NGC)) as a framework, an attenuated strain plasma of the DENV-2 (NGC)-poly (A) virus is obtained by deleting sequences from SLII to CS2 in a 3' untranslated region (3'UTR) of DENV-2 (NGC) (including SLII, RCS3, SLIV, CS3, DB1, RCS2, DB2 and CS2), retaining a cyclization sequence region 1 (CS1) and a short hairpin-3' stem loop region (sHP-3'SL) behind the cyclization sequence region 1 in the 3' untranslated region, and inserting a poly(A) sequence (140 adenylates) to a deletion site resulting from deletion of the sequences from SLI to CS2, and a nucleotide sequence of the attenuated strain plasma is shown in SEQ ID NO. 3.

Using a plasmid DNA from an infectious clone of a wild-type (WT) yellow fever virus (YFV) strain as a framework, an attenuated strain plasma of the YFV-poly (A) virus is obtained by deleting sequences from SLII to CS2 in a 3' untranslated region (3'UTR) of YFV (including SLII, RCS3, and DB), retaining a stem loop region (SL), a cyclization sequence region 1 (CS1) and a short hairpin-3' stem loop region (sHP-3'SL) behind the cyclization sequence region 1 in the 3' untranslated region, and inserting a poly(A) sequence (130 adenylates) to a deletion site resulting from deletion of the sequences from SLI to CS2, and a nucleotide sequence of the attenuated strain plasma is shown in SEQ ID NO. 4.

The plasmids of the above strains are all obtained by replacing all or a part of the 5'-end stem loop region (SL) sequence and all of the DB sequence in the 3'UTR region with the poly(A) sequence, retaining at least the cyclization sequence region 1 (CS 1) and the short hairpin-3' stem loop region (sHP-3'SL) behind the cyclization sequence region 1 in the 3' untranslated region, thereby obtaining the corresponding poly(A) virus attenuated strain plasmids.

### 2. In vitro transcription of Poly(A) into RNA

The plasmid of the attenuated strain of the poly(A) virus obtained in step 1 is linearized, after complete linearization is identified by 0.8% agarose gel electrophoresis, extracted with phenolic chloroform, and finally added with 11 µL of RNAase-free water to dissolve; and the concentration of DNA is determined using Thermo Scientific NanoDrop 2000 and the quality of DNA is detected by electrophoresis. Taking1 µg of DNA extracted with phenolic chloroform as a template, an in vitro transcription kit T7 mMESSAGE mMACHINE kit (Ambion) is used, an in vitro transcription is performed according to kit instructions to obtain a recombinant poly(A) RNA, namely a genomic RNA of attenuated strains of WNV-poly(A), JEV-poly(A), DENV-2(NGC)-poly(A) and YFV-poly(A) viruses. The concentration of RNA is determined using Thermo Scientific NanoDrop 2000 and the quality of RNA is detected by electrophoresis with 0.8% freshly prepared agarose gel, and the RNA is stored at -80°C for later use.

### 3. Poly(A) virus rescue

The experiment is divided into the following two groups:
(1) experimental group: a recombinant poly(A) RNA obtained by in vitro transcription in step 2 is transfected into BHK-21 cells by means of liposome transfection; and
(2) control group: a wild-type virus RNA is transfected into BHK-21 cells by means of liposome transfection as a positive control.

The specific experimental steps are as follows: on the day before transfection, 2×10⁵ BHK-21 cells are inoculated into 35 mm cell culture dishes, with three 10 mm × 10 mm coverslips being placed in each dish, such that about 80% of the cells are transfected on the day of transfection; duration transfection, medium in each dish is discarded, and the dish is washed once with 1 ml of Opti-MEM, and added with 1 ml of Opti-MEM (to keep the cells in an infiltrated state); 1 ml of Opti-MEM is added to a 1.5 ml EP tube, added with 4 µl of DMRIE-C (mixed well before use of DMRIE-C) and mixed well upside down, 1 µg of recombinant poly(A) RNA obtained by in vitro transcription is added to the experimental group respectively, and 1 µg of wild-type virus RNA is added into the control group and mixed well upside down; 1 µg of wild-type virus RNA is added to the control group and mixed well upside down; Opti-MEM in the dish is quickly discarded and the mixture is added to the dish softly (do not blow or bit the cells); and after incubation in a 37°C, CO₂ incubator for 4 h, the culture is discarded, and 2 mL of DMEM medium containing 2% FBS is added to each dish. A cell status of the experimental group and a cell status of the control group are observed under a microscope, and a glass slide is respectively taken 24, 48 and 72 h after transfection, and the cells are immobilized with a 5% acetone immobilization solution (purchased from Sinopharm Chemical Reagent Company); the cells are immobilized at room temperature for 15 minutes, washed with PBS three times, and stored at 4°C; when a positive rate of IFA detection is high, supernatants are respectively collected as poly(A) PO-generation viruses of attenuated flaviviruses, that is, four PO-generation attenuated WNV-poly(A), JEV-poly(A), DENV-2(NGC)-poly(A) and YFV-poly(A) strains are obtained and stored at -80°C.

### 4. Indirect immunofluorescence (IFA) to detect virus protein expression of recombinant poly(A)

The slide stored at 4°C in step 3 above is incubated at room temperature to obtain a primary antibody, and the attenuated flavivirus strain antibody is a 1:500-fold diluted 4G2 monoclonal antibody (general flavivirus E protein antibody). The primary antibody is incubated for 1-2 h at room temperature, and rinsed with PBS for10 times, and a secondary antibody is incubated at room temperature in the dark, wherein the secondary antibody is a 1:125-fold diluted conjugated fluorescein isothiocyanate (FITC) sheep anti-mouse antibody. After 1 h of incubation, the secondary antibody is rinsed with PBS for 10 times, the slide is taken out and marked. At each marker, 95% glycerol is dispensed. The coverslip with is placed on glycerol droplets with the side containing the cells facing downward, and observed under a fluorescence microscope at a 200 × magnification (FIG. 1).

The results in FIG. 1 show that the positive rate of each of attenuated WNV-poly(A), JEV-poly(A), DENV-2(NGC)-poly(A) and YFV-poly(A) strains at each time point is lower than that of WT, but with the prolongation of incubation time after transfection, the number of positive cells gradually increases, which is consistent with the trend of WT, indicating that WNV-poly(A), JEV-poly(A), DENV-2(NGC)-poly(A) and YFV-poly(A) all rescue infectious virus particles.

**Example 2:** Construction of other attenuated West Nile virus strain clone and virus rescue 1. Construction of other attenuated West Nile virus strain clone A schematic diagram of the other attenuated West Nile virus strain clone is shown in FIG. 2.

Using a plasmid DNA from an infectious clone of a wild-type (WT) West Nile virus (WNV) strain in the example 1 as a framework, an attenuated strain plasma of the West Nile virus SL-del-poly (A) is obtained by deleting sequences from SLI to CS3 in a 3' untranslated region (3'UTR) of MNV (including SLI, SLII, RCS3, SLIII, SLIV, and CS3), retaining a dumbbell region 1 (DB 1), a repetitive cyclization sequence region 2 (RCS2), a dumbbell region 2 (DB2), a cyclization sequence region 2 (CS2), a cyclization sequence region 1 (CS1) and a short hairpin-3' stem loop region (sHP-3'SL) behind the cyclization sequence region 1 in the 3' untranslated region, and inserting a poly(A) sequence (130 adenylates) to a deletion site resulting from deletion of the sequences from SLI to CS3 to obtain a plasmid of attenuated strain of West Nile virus SL-del-poly(A) deleted in the stem loop region (SL).

Using a plasmid DNA from an infectious clone of a wild-type (WT) West Nile virus (WNV) strain in the example 1 as a framework, an attenuated strain plasma of the West Nile virus DB-del-poly (A) from which a dumbbell region (DB) is deleted is obtained by deleting sequences from DBI to CS1 in a 3' untranslated region (3'UTR) of MNV (including DB 1, RCS2, DB2, CS2 and CS 1), retaining a 5'-end stem loop region I (SLI), a stem loop region II (SLII), a repetitive cyclization sequence region 3 (RCS3), a stem loop region III (SLIII), a stem loop region IV (SLIV), a cyclization sequence region 3 (CS3) and a short hairpin-3' stem loop region (sHP-3'SL) in the 3' untranslated region, and inserting a poly(A) sequence (130 adenylates) to a deletion site resulting from deletion of the sequences from DB 1 to CS1 to obtain a plasmid of attenuated strain of West Nile virus DB-del-poly(A) deleted in the dumbbell region (DB).

### 2. In vitro transcription of Poly(A) into RNA

The transcription method is described in step 2 of Example 1.

### 3. Poly(A) virus rescue

The rescue method is described in step 3 of Example 1.

### 4. Indirect immunofluorescence (IFA) to detect virus protein expression of recombinant poly(A)

The detection method is described in step 4 of Example 1.

The observation results of the fluorescence microscope are shown in FIG. 2. Results in FIG. 2 show that the virus obtained by deleting a part of the nucleotide sequence of the 3' untranslated region (3'UTR) of the flavivirus virus, without missing all 5'SL and DB, and inserting the poly(A) sequence to the deletion site, can also produce toxins.

### Example 3: Comparison of growth curve of attenuated flavivirus strain and growth curve of wild-type virus

### 1. Virus titer determination

The titers of the virus are determined by plaque phagocytosis according to the follow specific steps:
1×10⁵ BHK-21 cells are inoculated in each well of a 24-well cell culture plate, and when the cell confluency reaches 90%, a medium in the wells is discarded; and100 µl of poly(A) PO-generation virus of an attenuated flavivirus, which is collected with a 10-fold diluted DMEM medium containing 2% FBS in step 3 of Example 1, is added, adsorbed at 37°C for 1 h, and shaken well every 15 min. After adsorption, the virus liquid of each well is discarded by suction, a DMEM medium containing 2% FBS and a covering of 2% methylcellulose are added, and inoculated in a 37°C, 5% CO₂ incubator for 3 days, stained with a staining solution containing 1% crystal violet and 3.7% formaldehyde after the formation of plaques, and treated at room temperature for 30 min; and the staining solution in the wells are taken out, the bottoms of the wells are rinsed with running water and dried, and then the virus titers are obtained by counting and conversion.

### 2. Determination of growth curves of viruses

2×10⁶ Vero cells are inoculated in 35 mm cell culture dishes; under the culture conditions at 37 °C and in 5% CO₂, when the confluency reaches 60%, the poly(A) PO-generation virus collected in step 3 of Example 1 and the corresponding wild-type (WT) virus are added to each dish according to the multiplicity of infection MOI 0.1, and adsorbed in a 37°C, 5% CO₂ incubator for 2 h, and then the virus liquid is discarded; 2 ml of DMEM medium containing 2% fetal bovine serum is added to each well and incubated under the incubation conditions at 37°C and in 5% CO₂; 400 µl of virus supernatant is collected and supplemented with 400 µl of DMEM medium containing 2% fetal bovine serum every 12 h after infection; and the collected viruses are used as virus samples at different time points under the same multiplicity of infection between the poly(A) virus and the corresponding WT virus, and stored at -80°C. The viral titers collected at different time points are determined according to the plaque assay described above and the growth curves are plotted (FIG. 3). The results show that attenuated WNV-poly(A), JEV-poly(A), DENV-2(NGC)-poly(A) and YFV-poly(A) strains have similar growth trends compared with the corresponding WT, while the titers thereof are each 10-100 times lower than that of the wild type.

### Example 4: Verification of genetic stability of attenuated flavivirus strains

1. The poly(A) PO-generation virus of the attenuated flavivirus obtained by rescue in Example 1 is used to infect Vero cells, and after 3 days, the cytopathic effect (CPE) is obvious; a cellular supernatant (P1 generation) is collected, and three virus strains are passaged in parallel in each generation, and blindly passaged for 10 consecutive passages; and an RNA of the virus is extracted from virus-infected cell samples collected in the P1, P5, and P10 generations according to the instructions of a Trizol kit, and stored at -80°C for later use. RT-PCR is performed with PrimeScript One Step RT-PCR Kit to detect changes in 3'UTR, followed by sequencing. The sequencing results show that WNV-poly(A), JEV-poly(A), DENV-2(NGC)-poly(A), and YFV-poly(A) are continuously passaged for 10 generations, while polyA insertion locations and number remained essentially unchanged, about 130-150 A (FIG. 4).
2. The virus supernatant collected by P1 and P10 generations is taken for comparison of the growth curves in Vero cells according to the multiplicity of injection MOI of 0.1 (the method is the same as Example 2); and the results are shown in poly(A)-P10 markers in FIG. 4, indicating that the growth curves of WNV-poly(A), JEV-poly(A), DENV-2(NGC)-poly(A) and YFV-poly(A) virus strains in respective P1 and P10 generations are similar, further indicating that the above poly(A) virus has good genetic stability.

### Example 5: Pathogenicity of attenuated West Nile virus

This example investigates the pathogenicity of attenuated West Nile virus (WNV) strain WNV-poly(A) on C57BL/6.

Six groups of 4-week-old female C57BL/6 mice (5 mice/group) are taken and infected intraperitoneally with 10⁴ PFU, 10⁵ PFU, 10⁶ PFU or 10⁷ PFU of P1-generation attenuated WNV-poly(A) strain obtained in Example 3 and 10⁴ PFU of corresponding wild-type West Nile virus (WT WNV), and mice injected with equal volume of PBS are used as controls. On Days 1, 2, and 3 after infection, blood toxicity determination is performed by detecting the virus titers in the serum of mice. The specific method includes: collecting about 100 µl of blood from the orbit of each mouse and putting it in a 1.5 ml EP tube, standing at 4°C for 3 h, then collecting the serum by centrifugation at 5000 rpm for 5 min for virus titer determination. The determination regimen is the same as the virus titer determination in Example 1. The mice are weighed for 14 consecutive days after simultaneous infection and the survival statuses of the mice are observed within 21 days.

The results show that the mice inoculated with the attenuated WNV-poly(A) strain all survive (FIG. 5A), and no viremia is detected in other doses except for the mice inoculated with 10⁷ PFU of attenuated WNV-poly(A) strain being detected to have slight viremia on the first day (B in FIG. 5); and the changes in body weight are consistent with the changes in a PBS immune group (C in FIG. 5) and no other pathogenesis is observed. However, the mice inoculated with 10⁴ PFU of WT WNV manifest hyper viremia (B in FIG. 5), with significant weight loss from Day 5 (C in FIG. 5), and all die on Day 8 (A in FIG. 5). It is shown that the attenuated WNV-poly(A) strain has sufficiently reduced virulence, does not cause a disease in the C57BL/6 mice, has high safety, and can be used as a potential live attenuated vaccine.

### Example 6: Humoral immunity and challenge protection effects of attenuated West Nile virus in mouse model

This example investigates the humoral immunity and challenge protection effects of an attenuated WNV-poly(A) strain in a C57BL/6 mouse model.
1.The humoral immunity of the attenuated WNV-poly(A) strain in a C57BL/6 mouse model

Six groups of 4-week-old female C57BL/6 mice (5 mice/group) are taken and infected intraperitoneally with 10⁴ PFU, 10⁵ PFU, 10⁶ PFU or 10⁷ PFU of attenuated P1-generation WNV-poly(A) strain obtained in Example 3, and mice in a Mock group which are immunized with equal volume of PBS and the mice in the Mock group which are not treated are used as controls. On Days 14 and 28 after immunization, blood is collected according to an orbital blood collection method in Example 4, and serum is collected by centrifugation after standing at 4°C for 3 h, inactivated at 56°C for 30 min, and frozen at -20°C for later use.

IgG antibody titers are detected by ELISA and neutralizing antibody titers are detected by PRNT50, respectively:

### 1) Detection of IgG antibody titers by ELISA

A 96-well plate is coated with an attenuated WNV-poly(A) strain and blocked with 5% skim milk prepared from PBS; serum is subjected to 4-fold serial dilution from 1:50 and then incubated with the coated 96-well plate at 37°C for 2 h; after the plate is washed with PBST for three times, an HRP sheep anti-mouse antibody is incubated at 37°C for 1 h, and developed with a two-component chromogenic kit (Proteintech); after the addition of 1 M H₂SO₄ for termination, an absorbance value at 450 nm is detected with a multifunctional microplate reader; and the IgG antibody titer is the highest dilution when the serum absorbance value of immunized mice is 2 times the serum absorbance value of unimmunized mice.

### 2) Detection of neutralizing antibody titers by PRNT50

2 × 10⁵ BHK-21 cells are inoculated in each well of a 12-well cell culture plate, respectively; when the cell confluency reaches 90%, the serum is first subjected to 2-fold serial dilution; 100 µl of diluted antibody and an equal volume of 100 PFU of WT-WNV are well mixed and incubated at 37°C for 1 h; the medium in the wells is discarded, and the incubated serum-virus mixture is added to the corresponding wells, adsorbed at 37°C for 1 h, and shaken well every 15 min; after adsorption, the virus solution in each well is discarded by suction, 1 mL of covering containing 2% methylcellulose is added, incubated in an 37°C, 5% CO₂ incubator for 72 h, stained with a staining solution containing 1% crystal violet and 3.7% formaldehyde after the formation of plaques, and treated at room temperature for 30 min; the staining solution is removed from the wells and the bottoms of the wells are rinsed with running water, and after drying, the virus titers after serum neutralization can be counted at each dilution; and the neutralizing antibody titer (PRNT50) in serum is the highest dilution of serum when the virus titer is neutralized by 50%.

The results show that C57BL/6 mice immunized with different doses of attenuated WNV-poly(A) strains produce a strong antibody response after 14 days, and the IgG antibody titer and neutralizing antibody titer are increased at Day 28 after immunization. The mice immunized with different doses produce specific IgG antibody titers without significant dose-dependence, and IgG antibody titers are all around 1:12800 at Day 28 after immunization (FIG. 6A). Neutralizing antibody levels increase with increasing immunization dose, wherein the neutralizing antibody titer of mice immunized with10⁴ PFU of attenuated WNV-poly(A) strain after 28 days is 1:40-1:80, and the antibody titer of mice immunized with10⁷ PFU of attenuated WNV-poly(A) strain is 1:160-1:640 (B in FIG. 6). It is demonstrated that the attenuated WNV-poly(A) strain can produce a strong antibody response against WNV after immunizing the C57BL/6 mice.

### 2. Challenge protection effects of attenuated WNV-poly(A) strain in C57BL/6 mouse model

On Day 30 after immunization, except for the Mock group which is not subjected to any treatment, the remaining five groups of mice are subjected to intraperitoneal challenge with 10⁷ PFU of WT WNV. The mice are recorded for the survival status daily and weighed, and the blood toxicity is determined in accordance with the method in Example 4 on Day 2 after challenge. The results show that the control group mice immunized with PBS begin to lose weight significantly on Day 5 after the challenge (D in FIG. 6), and high blood toxicity levels are detected on Day 2 after challenge (E in FIG. 6), showing symptoms such as hair frizzy, hindlimb paralysis, etc., and all died on Day 9 (C in FIG. 6). The mice that are immunized with the attenuated WNV-poly(A) strain all survive, which is similar to the mice in the Mock group without any treatment, so there are no any onset symptoms and any blood toxicity are detected. The above experiments prove that the attenuated WNV-poly(A) strain can provide good immune protection against C57BL/6 mice as an attenuated vaccine.

### Example 7: CD8⁺T cell response of attenuated West Nile virus in mouse model

This example investigates a CD8⁺ T cell response of an attenuated WNV-poly (A) strain in a C57BL/6 mouse model,
The CD8⁺ T cell response plays an important role against a West Nile virus (WNV) infection. In order to detect whether the attenuated WNV-poly(A) strain can induce a CD8⁺ T cell response after immunizing mice, two groups of 6- to 8-week-old female C57BL/6 mice (3 mice/group) are taken, wherein the mice in one group are intraperitoneally immunized with 10⁷ PFU of attenuated WNV-poly(A) strain, while the mice in the other group are immunized with an equal volume of PBS as a control. The spleen of each mouse is harvested on Day 7 after immunization and subjected to in vitro stimulation with specific polypeptides (WNV E protein and NS4B protein), while the spleen of a mouse subjected to in vitro stimulation with non-specific stimulants (phorbol myristate acetate (PMA) and ionomycin (IONO)) is used as a positive control; and the proliferation of CD8⁺ IFN-γ⁺ T cells is detected by an ELISPOT method. The results are shown in FIG. 7. The CD8⁺ IFN-γ⁺ T cells of the mice immunized with the attenuated WNV-poly(A) strain show significant activation under the specific peptides and the non-specific stimulants, while the mice in the Mock group do not have any CD8⁺ IFN-γ⁺ T cell activation under specific peptide stimulation. The above results show that the attenuated WNV-poly(A) strain rescued by the present disclosure can produce a strong CD8⁺ IFN-γ⁺ T cell response against WNV E protein and NS4B epitope properties after immunizing with C57BL/6 mice.

### Example 8: Long-term immune protection effects of attenuated West Nile virus in mouse model

This example investigates the long-term immune protection effects of an attenuated WNV-poly(A) strain on C57BL/6.

Three groups of 4-week-old female C57BL/6 mice (5 mice/group) are taken and infected intraperitoneally with 10⁴ PFU and 10⁷ PFU of attenuated WNV-poly(A) strain respectively, and mice immunized with PBS are used as controls. After immunization, orbital blood is collected at Day 28, Day 56, Day 112 and Day 168, and specific IgG and neutralizing antibody titers are detected according to the method in Example 5. On Day 170 after immunization, the mice are infected intraperitoneally with 10⁷ PFU of WN WNV for a challenge experiment, and on Day 2 after the challenge, the blood toxicity is determined according to the method in Example 4 and the survival rate of mice is determined. The results show that the specific IgG antibody and neutralizing antibody titers caused by mice immunized with 10⁴ PFU and 10⁷ PFU of attenuated WNV-poly(A) strain remain basically stable within 168 days (A and B in FIG. 8), the survival rate of the mice after challenge is 100%, and no blood toxicity is detected on Day 2 after challenge (C and D in FIG. 8). The above experiment proves that the attenuated WNV-poly(A) strain can provide long-term immune protection for the mice.

### Example 9: Pathogenicity of attenuated dengue virus

This example investigates the pathogenicity of attenuated dengue virus (DENV) strain DENV-poly(A) on AG129.

Five groups of 4-week-old female AG129/6 mice (5 mice/group) are taken and infected intraperitoneally with 10³ PFU, 10⁴ PFU or 10⁵ PFU of attenuated DENV-poly(A) strain and 10⁴ PFU of WT DENV, and mice injected with equal volume of PBS are used as controls. On Days 2, and 3 after infection, blood toxicity determination is performed by detecting the virus titers in the serum of mice. The specific method includes: collecting about 100 µl of blood from the orbit of each mouse and putting it in a 1.5 ml EP tube, standing at 4°C for 3 h, then collecting the serum by centrifugation at 5000 rpm for 5 min for virus titer determination. The determination scheme is the same as the virus titer determination in Example 1. The mice are weighed for 27 consecutive days after simultaneous infection and the survival statuses of the mice are observed within 27 days.

The results show that the mice inoculated with the attenuated DENV-poly(A) strain all survive (A in FIG. 9), and no viremia is detected in other doses (B in FIG. 9); and the changes in body weight are consistent with the changes in a PBS immune group (C in FIG. 9) and no other pathogenesis is observed. However, the mice inoculated with 10⁴ PFU of WT DENV manifest hyper viremia (B in FIG. 9), with significant weight loss from Day 10 (C in FIG. 9), and all die on Day 21 (A in FIG. 9). It is shown that the attenuated DENV-poly(A) strain has sufficiently reduced virulence, does not cause a disease in the AG129 mice, has high safety, and can be used as a potential live attenuated vaccine.

### Example 10: Humoral immunity and challenge protection effects of attenuated dengue virus in mouse model

This example investigates the humoral immunity and challenge protection effects of an attenuated DENV-poly(A) strain in a AG129 mouse model.

### 1. The humoral immunity of attenuated DENV-poly(A) strain in AG129 mouse model

Five groups of 4-week-old female AG129 mice (5 mice/group) are taken and infected intraperitoneally with 10³ PFU, 10⁴ PFU or 10⁵ PFU of attenuated P1-generation DENV-poly(A) strain obtained in Example 3, and mice in a Mock group which are immunized with equal volume of PBS and the mice in the Mock group which are not treated are used as controls. On Days 28 after immunization, blood is collected according to an orbital blood collection method in Example 4, and serum is collected by centrifugation after standing at 4°C for 3 h, inactivated at 56°C for 30 min, and frozen at -20°C for later use.

IgG antibody titers are detected by ELISA and neutralizing antibody titers are detected by PRNT50, respectively:

### 1) Detection of IgG antibody titers by ELISA

A 96-well plate is coated with an attenuated DENV-poly(A) strain and blocked with 5% skim milk prepared from PBS; serum is subjected to 4-fold serial dilution from 1:50 and then incubated with the coated 96-well plate at 37°C for 2 h; after the plate is washed with PBST for three times, an HRP sheep anti-mouse antibody is incubated at 37°C for 1 h, and developed with a two-component chromogenic kit (Proteintech); after the addition of 1 M H₂SO₄ for termination, an absorbance value at 450 nm is detected with a multifunctional microplate reader; and the IgG antibody titer is the highest dilution when the serum absorbance value of immunized mice is 2 times the serum absorbance value of unimmunized mice.

### 2) Detection of neutralizing antibody titers by PRNT50

2 × 10⁵ BHK-21 cells are inoculated in each well of a 12-well cell culture plate, respectively; when the cell confluency reaches 90%, the serum is first subjected to 2-fold serial dilution; 100 µl of diluted antibody and an equal volume of 100 PFU of WT-DENV are well mixed and incubated at 37°C for 1 h; the medium in the wells is discarded, and the incubated serum-virus mixture is added to the corresponding wells, adsorbed at 37°C for 1 h, and shaken well every 15 min; after adsorption, the virus solution in each well is discarded by suction, 1 mL of covering containing 2% methylcellulose is added, incubated in an 37°C, 5% CO₂ incubator for 72 h, stained with a staining solution containing 1% crystal violet and 3.7% formaldehyde after the formation of plaques, and treated at room temperature for 30 min; the staining solution is removed from the wells and the bottoms of the wells are rinsed with running water, and after drying, the virus titers after serum neutralization can be counted at each dilution; and the neutralizing antibody titer (PRNT50) in serum is the highest dilution of serum when the virus titer is neutralized by 50%.

The results show that AG129 mice immunized with different doses of attenuated DENV-poly(A) strains in single immunization produce a strong antibody response after 28 days. The IgG antibody titers are all around 1:3200-1:12800 (A in FIG.10). Neutralizing antibody levels increase with increasing immunization dose, wherein the neutralizing antibody titer of mice immunized with 10³ PFU of attenuated WNV-poly(A) strain after 28 days is 1:40-1:80, and the neutralizing antibody titer of mice immunized with10⁵ PFU of attenuated WNV-poly(A) strain is 1:80-1:160 (B in FIG. 6). It is demonstrated that the attenuated DENV-poly(A) strain can produce a strong antibody response against DENV after immunizing the AG129 mice.

### 2. Challenge protection effects of attenuated DENV-poly(A) strain in AG129 mouse model

On Day 30 after immunization, except for the Mock group which is not subjected to any treatment, the remaining four groups of mice are subjected to intraperitoneal challenge with 10⁶ PFU of WT DENV. The mice is recorded for the survival daily and weighed, and the toxic blood is determined in accordance with the method in Example 4 on Day 3 after challenge. The results show that the control group mice immunized with PBS begin to lose weight significantly on Day 4 after the challenge (D in FIG. 10), and high blood toxicity levels are detected on Day 3 after challenge (E in FIG. 10), showing symptoms such as dorsal hair standing up, and all die on Day 16 (C in FIG. 10). The mice that are immunized with the attenuated DENV-poly(A) strain all survive, which is similar to the mice in the Mock group without any treatment, so no any onset symptoms and any blood toxicity are detected. The above experiment proves that the attenuated DENV-poly(A) strain can provide good immune protection against AG129 mice as an attenuated vaccine.

## Claims

1. An attenuated flavivirus virus, comprising a polyadenylic acid (poly(A)) sequence, wherein the polyadenylic acid (poly(A)) sequence is used for replacing a part of nucleotide sequence of a 3' untranslated region (3'UTR) of the flavivirus virus, so that the 3' untranslated region (3'UTR) of the attenuated flavivirus virus obtained in response to the part of the nucleotide sequence of the flavivirus virus being replaced at least retains a 3'-end stem loop region (3'SL).

2. The attenuated flavivirus virus according to claim 1, wherein the part of nucleotide sequence comprises all or a part of a nucleotide sequence of a 5'-end stem loop region (5'SL), all or a part of a nucleotide sequence of a cyclization sequence region (CS), and/or all or a part of a nucleotide sequence of a dumbbell region (DB) in a 3' untranslated region (3'UTR);
preferably, after the part of nucleotide sequence is replaced by the polyadenylic acid (poly(A)) sequence, the 3' untranslated region (3'UTR) of the attenuated flavivirus virus retains at least a cyclization sequence region 1 (CS1) and a short hairpin 3' stem loop region (sHP-3'SL) behind the cyclization sequence region 1 in the 3' untranslated region; preferably, the 5'-end stem loop region (5'SL) in the 3' untranslated region (3'UTR) is selected from a stem loop region I (SLI), a stem loop region II (SLII), a stem loop region III (SLIII) and a stem loop region IV (SLIV);
preferably, the cyclization sequence region (CS) is selected from a cyclization sequence region 1 (CS1), a cyclization sequence region 2 (CS2), a cyclization sequence region 3 (CS3), a repetitive cyclization sequence region 2 (RCS2) and a repetitive cyclization sequence region 3 (RCS3);
preferably, the dumbbell region (DB) is selected from a dumbbell region 1 (DB1) and a dumbbell region 2 (DB2).

3. The attenuated flavivirus virus according to claim 1 or 2, wherein the polyadenylic acid (poly(A)) sequence comprises 10-200 adenylates;
preferably, the polyadenylic acid (poly(A)) sequence comprises 50-180, 100-160 adenylates; more preferably, 130-150 adenylates, preferably 130 or 150 adenylates;
preferably, the flavivirus virus is selected from the group consisting of Japanese encephalitis virus (JEV), West Nile virus (WNV), tick-borne encephalitis virus (TBEV), yellow fever virus (YFV), dengue virus (DENV), zika virus (ZIKV), St. Louis encephalitis virus (SLEV), Murray Valley encephalitis virus (MVEV), Omsk hemorrhagic fever virus and Kyasanur Forest Disease virus;
preferably, the attenuated flavivirus virus has a plaque titer of 5×10⁴-5×10⁶ PFU/ml;
preferably, the attenuated flavivirus virus is 10-100 times less virulent than a parent wild-type virus;
preferably, the attenuated flavivirus virus is obtained by means of rescue on baby hamster kidney cells BHK-21 and/or amplification on African green monkey kidney Vero cells;
preferably, the dengue virus is selected from 1-type, 2-type, 3-type and 4-type dengue viruses;
preferably, the attenuated flavivirus virus is an attenuated West Nile virus; preferably, the attenuated West Nile virus comprises a nucleotide sequence having 80% or more identity with a nucleotide sequence shown in SEQ ID NO. 1, preferably a nucleotide sequence having more than 85%, 90%, 95%, 96%, 97%, 98%, 99% identity with the nucleotide sequence shown in SEQ ID NO. 1, more preferably a nucleotide sequence having 98% or 99% or more identity with the nucleotide sequence shown in SEQ ID NO. 1; more preferably, the nucleotide sequence of the attenuated West Nile virus is SEQ ID NO. 1;
preferably, the attenuated flavivirus virus is an attenuated Japanese encephalitis virus; preferably, the attenuated Japanese encephalitis virus comprises a nucleotide sequence having 80% or more identity with a nucleotide sequence shown in SEQ ID NO. 2, preferably a nucleotide sequence having more than 85%, 90%, 95%, 96%, 97%, 98%, 99% identity with the nucleotide sequence shown in SEQ ID NO. 2, more preferably a nucleotide sequence having 98% or 99% or more identity with the nucleotide sequence shown in SEQ ID NO. 2; more preferably, the nucleotide sequence of the attenuated Japanese encephalitis virus is SEQ ID NO. 2;
preferably, the attenuated flavivirus virus is an attenuated dengue virus; preferably, the attenuated dengue virus comprises a nucleotide sequence having 80% or more identity with a nucleotide sequence shown in SEQ ID NO. 3, preferably a nucleotide sequence having more than 85%, 90%, 95%, 96%, 97%, 98%, 99% identity with the nucleotide sequence shown in SEQ ID NO. 3, more preferably a nucleotide sequence having 98% or 99% or more identity with the nucleotide sequence shown in SEQ ID NO. 3; more preferably, the nucleotide sequence of the attenuated dengue virus is SEQ ID NO. 3;
preferably, the attenuated flavivirus virus is an attenuated yellow fever virus; preferably, the attenuated yellow fever virus comprises a nucleotide sequence having 80% or more identity with a nucleotide sequence shown in SEQ ID NO. 4, preferably a nucleotide sequence having more than 85%, 90%, 95%, 96%, 97%, 98%, 99% identity with the nucleotide sequence shown in SEQ ID NO. 4, more preferably a nucleotide sequence having 98% or 99% or more identity with the nucleotide sequence shown in SEQ ID NO. 4; more preferably, the nucleotide sequence of the attenuated yellow fever virus is SEQ ID NO. 4;
preferably, the attenuated flavivirus virus is an attenuated zika virus; preferably, the attenuated zika virus comprises a nucleotide sequence having 80% or more identity with a nucleotide sequence shown in SEQ ID NO. 5, preferably a nucleotide sequence having more than 85%, 90%, 95%, 96%, 97%, 98%, 99% identity with the nucleotide sequence shown in SEQ ID NO. 5, more preferably a nucleotide sequence having 98% or 99% or more identity with the nucleotide sequence shown in SEQ ID NO. 5; more preferably, the nucleotide sequence of the attenuated zika virus is SEQ ID NO. 5;
preferably, the attenuated flavivirus virus is an attenuated tick-borne encephalitis virus; preferably, the attenuated tick-borne encephalitis virus comprises a nucleotide sequence having 80% or more identity with a nucleotide sequence shown in SEQ ID NO. 6, preferably a nucleotide sequence having more than 85%, 90%, 95%, 96%, 97%, 98%, 99% identity with the nucleotide sequence shown in SEQ ID NO. 6, more preferably a nucleotide sequence having 98% or 99% or more identity with the nucleotide sequence shown in SEQ ID NO. 6; more preferably, the nucleotide sequence of the attenuated tick-borne encephalitis virus is SEQ ID NO. 6;
preferably, the part of nucleotide sequence comprises a 5'-end stem loop region I (SLI), a stem loop region II (SLII), a repetitive cyclization sequence region 3 (RCS3), a stem loop region III (SLIII), a stem loop region IV (SLIV), and a cyclization sequence region 3 (CS3), and after the part of nucleotide sequence is replaced by the polyadenylic acid (poly(A)) sequence, the 3' untranslated region (3'UTR) of the attenuated flavivirus virus retains a dumbbell region 1 (DB1), a repetitive cyclization sequence region 2 (RCS2), a dumbbell region 2 (DB2), a cyclization sequence region 2 (CS2), a cyclization sequence region 1 (CS1) and a short hairpin 3' stem loop region (sHP-3'SL) behind the cyclization sequence region 1 in the 3' untranslated region;
preferably, the part of nucleotide sequence comprises a dumbbell region 1 (DB1), a repetitive cyclization sequence region 2 (RCS2), a dumbbell region 2 (DB2), a cyclization sequence region 2 (CS2), and a cyclization sequence region 1 (CS1), and after the part of nucleotide sequence is replaced by the polyadenylic acid (poly(A)) sequence, the 3' untranslated region (3'UTR) of the attenuated flavivirus virus retains a 5'-end stem loop region I (SLI), a stem loop region II (SLII), a repetitive cyclization sequence region 3 (RCS3), a stem loop region III (SLIII), a stem loop region IV (SLIV), a cyclization sequence region 3 (CS3) and a short hairpin 3' stem loop region (sHP-3'SL) behind the cyclization sequence region 1 in the 3' untranslated region;
preferably, the part of nucleotide sequence comprises a 5'-end stem loop region I (SLI), a stem loop region II (SLII), a repetitive cyclization sequence region 3 (RCS3), a stem loop region III (SLIII), a stem loop region IV (SLIV), a cyclization sequence region 3 (CS3), a dumbbell region 1 (DB1), a repetitive cyclization sequence region 2 (RCS2), a dumbbell region 2 (DB2), and a cyclization sequence region 2 (CS2), and after the part of nucleotide sequence is replaced by the polyadenylic acid (poly(A)) sequence, the 3' untranslated region (3'UTR) of the attenuated flavivirus virus retains a cyclization sequence region 1 (CS1) and a short hairpin 3' stem loop region (sHP-3'SL) behind the cyclization sequence region 1 in the 3' untranslated region;
preferably, the part of nucleotide sequence comprises a 5'-end stem loop region II (SLII), a repetitive cyclization sequence region 3 (RCS3), a stem loop region IV (SLIV), a cyclization sequence region 3 (CS3), a dumbbell region 1 (DB 1), a repetitive cyclization sequence region 2 (RCS2), a dumbbell region 2 (DB2), and a cyclization sequence region 2 (CS2), and after the part of nucleotide sequence is replaced by the polyadenylic acid (poly(A)) sequence, the 3' untranslated region (3'UTR) of the attenuated flavivirus virus retains a cyclization sequence region 1 (CS1) and a short hairpin 3' stem loop region (sHP-3'SL) behind the cyclization sequence region 1 in the 3' untranslated region;
preferably, the part of nucleotide sequence comprises a 5'-end stem loop region I (SLII), a repetitive cyclization sequence region 3 (RCS3), a dumbbell region (DB), a repetitive cyclization sequence region 2 (RCS2), a dumbbell region 2 (DB2), and a cyclization sequence region 2 (CS2), and after the part of nucleotide sequence is replaced by the polyadenylic acid (poly(A)) sequence, the 3' untranslated region (3'UTR) of the attenuated flavivirus virus retains 5'-end stem loop region (SL), a cyclization sequence region 1 (CS1) and a short hairpin 3' stem loop region (sHP-3'SL) behind the cyclization sequence region 1 in the 3' untranslated region.

4. A DNA, which can be transcribed to produce an RNA genome of the attenuated flavivirus virus according to any one of claims 1 to 3, wherein
preferably, the DNA is an infectious clone of an attenuated strain of the flavivirus virus, preferably the infectious clone is a plasmid.

5. A cell, comprising the attenuated flavivirus virus according to any one of claims 1 to 3 or the DNA according to claim 4.

6. A vaccine, comprising the attenuated flavivirus virus according to any one of claims 1 to 3, the DNA according to claim 4, and/or the cell according to claim 5.

7. A pharmaceutical composition, comprising the attenuated flavivirus virus according to any one of claims 1 to 3, the DNA according to claim 4, the cell according to claim 5 and/or the vaccine according to claim 6, and a pharmaceutically acceptable vector.

8. A method for preparing the attenuated flavivirus virus according to any one of claims 1 to 3, the method comprising substituting a part of nucleotide sequence of a 3' untranslated region (3'UTR) of a wild-type virus with a polyadenylic acid (poly(A)) sequence, wherein preferably, the attenuated flavivirus virus is obtained by means of rescue on baby hamster kidney cells BHK-21 and/or amplification on African green monkey kidney Vero cells.

9. Use of the attenuated flavivirus virus according to any one of claims 1 to 3, the DNA according to claim 4 and the cell according to claim 5 in the preparation of an attenuated flavivirus virus vaccine, wherein
preferably, the use is use of the attenuated West Nile virus in the preparation of an attenuated West Nile virus vaccine;
preferably, the use is use of the attenuated Japanese encephalitis virus in the preparation of an attenuated Japanese encephalitis virus vaccine;
preferably, the use is use of the attenuated dengue virus in the preparation of an attenuated dengue virus vaccine;
preferably, the use is use of the attenuated yellow fever virus in the preparation of an attenuated yellow fever virus vaccine;
preferably, the use is use of the attenuated zika virus in the preparation of an attenuated zika virus vaccine.

10. Use of the attenuated flavivirus virus according to any one of claims 1 to 3, the DNA according to claim 4, the cell according to claim 5, the vaccine according to claim 6 and/or the pharmaceutical composition according to claim 7 in the preparation of a medicament for treating or preventing diseases caused by a flavivirus virus, wherein
preferably, the use is use of the attenuated West Nile virus in the preparation of a medicament for treating or preventing diseases caused by the West Nile virus;
preferably, the use is use of the attenuated Japanese encephalitis virus in the preparation of a medicament for treating or preventing diseases caused by the Japanese encephalitis virus;
preferably, the use is use of the attenuated dengue virus in the preparation of a medicament for treating or preventing diseases caused by the dengue virus;
preferably, the use is use of the attenuated yellow fever virus in the preparation of a medicament for treating or preventing diseases caused by the yellow fever virus;
preferably, the use is use of the attenuated zika virus in the preparation of a medicament for treating or preventing diseases caused by the zika virus.
